# EUROPEAN PATENT SPECIFICATION

(11) **EP 1 608 262 B1**
(45) Date of publication and mention of the grant of the patent: **05.06.2013**
(21) Application number: 04749353.1
(22) Date of filing: 05.03.2004
(51) Int. Cl.: A61B 5/00, A61B 5/05

(54) **APPARATUS FOR TRACKING INSERTION DEPTH**
GERÄT ZUR VERFOLGUNG DER EINFÜHRUNGSTIEFE
DISPOSITIF DE DETECTION DE PROFONDEUR D'INSERTION

(30) Priority: 07.03.2003 US 384252
(43) Date of publication of application: 28.12.2005
(60) Divisional application: 10004306.6
(73) Proprietor: Intuitive Surgical Operations, Inc., Sunnyvale CA 94086 (US)
(72) Inventor: WHITIN, Katherine, Woodside, CA 94062 (US); OHLINE, Robert, M., Redwood City, CA 94061 (US); BELSON, Amir, Los Altos, CA 94024 (US); ROTH, Alex, Redwood City, CA 94061 (US); ARNE, Lawrence, W., Redwood City, CA 94062 (US)
(74) Representative: Price, Nigel John King
(86) International application number: PCT/US2004/006939
(87) International publication number: WO 2004/084702

(56) References cited:
- EP-A- 0 993 804
- WO-A-00/10456
- WO-A-95/09562
- WO-A-96/05768
- WO-A-97/25101
- WO-A-97/29701
- WO-A-97/29710
- WO-A-98/49938
- US-A- 6 063 022
- US-B1- 6 185 448
- US-B2- 6 610 007

## Description

### FIELD OF THE INVENTION

The present invention relates generally to endoscopes and endoscopic medical procedures. More particularly, it relates to apparatus for tracking the insertion and/or withdrawal of a flexible endoscope along a tortuous path, such as for colonoscopic examination and treatment.

### BACKGROUND OF THE INVENTION

An endoscope is a medical instrument for visualizing the interior of a patient's body. Endoscopes can be used for a variety of different diagnostic and interventional procedures, including colonoscopy, bronchoscopy, thoracoscopy, laparoscopy and video endoscopy.

Colonoscopy is a medical procedure in which a flexible endoscope, or colonoscope, is inserted into a patient's colon for diagnostic examination and/or surgical treatment of the colon. A standard colonoscope is typically 135-185 cm in length and 12-19 mm in diameter, and includes a fiberoptic imaging bundle or a miniature camera located at the instrument's tip, illumination fibers, one or two instrument channels that may also be used for insufflation or irrigation, air and water channels, and vacuum channels. The colonoscope is usually inserted via the patient's anus and advanced through the colon, allowing direct visual examination of the colon, the ileocecal valve and portions of the terminal ileum. Insertion of the colonoscope is complicated by the fact that the colon represents a tortuous and convoluted path. Considerable manipulation of the colonoscope is often necessary to advance the colonoscope through the colon, making the procedure more difficult and time consuming and adding to the potential for complications, such as intestinal perforation. Steerable colonoscopes have been devised to facilitate selection of the correct path though the curves of the colon. However, as the colonoscope is inserted farther and farther into the colon, it becomes more difficult to advance the colonoscope along the selected path. At each turn, the wall of the colon must maintain the curve in the colonoscope. The colonoscope rubs against the mucosal surface of the colon along the outside of each turn. Friction and slack in the colonoscope build up at each turn, making it more and more difficult to advance and withdraw the colonoscope. In addition, the force against the wall of the colon increases with the buildup of friction. In cases of extreme tortuosity, it may become impossible to advance the colonoscope all of the way through the colon.

Another problem which arises, for example, in colonoscope procedures, is the formation of loops in the long and narrow tube of the colonoscope. Such loops may arise when the scope encounters an obstacle, or gets stuck in a narrow passage. Instead of progressing, the scope forms loops within the patient. In an attempt to proceed in insertion of the colonoscope, excess force may be exerted, damaging delicate tissue in the patient's body. The physician may proceed with the attempted insertion of the endoscope without realizing there is a problem.

Through a visual imaging device the user can observe images transmitted from the distal end of the endoscope. From these images and from knowledge of the path the endoscope has followed, the user can ordinarily determine the position of the endoscope. However, it is difficult to determine the endoscope position within a patient's body with any great degree of accuracy. This becomes even more difficult when attempting to determine endoscopic positioning using, e.g., automatically controlled endoscopic devices, as described in U.S. Pat. No. 6,468,203; U.S. Pat. App. No. 09/969,927 filed October 2, 2001; U.S. Pat. App. No. 10/229,577 filed August 27,2002; U.S. Pat. App. No. 10/087,100 filed March 1, 2002; and U.S. Pat. App. No. 10/139,289 filed May 2, 2002.

Another method used to determine the configuration of the endoscope is x-ray imaging. Yet another method used is magnetic field positioning, which avoids the x-ray exposure to the patient and the operator. Such a method typically uses magnetic position determination via low frequency magnetic fields to determine the position of a miniature sensor embedded within the endoscope tube. Based on the position of the sensor at sequential time periods, an image of the configuration of the endoscope tube is produced.

Another method involves the placement of a series of markings on the endoscope that can aid the physician in proper placement of the device in the patient's body during a procedure. These markings can include bands, dots, lettering, numbering, colors, or other types of indicia to indicate position or movement of the device within the body. Visually distinguishable marks are often located at regular predetermined intervals. Such a system of indicia can be made to be visible under fluoroscopy by the use of certain radiopaque metals, or compounds incorporated into or printed on the device.

However, each of these methods are limited in their flexibility and applicability when the position of the endoscope within a patient's body is desired with any accuracy. Furthermore, such conventional position determination methods in many cases may also fail to account for the real-time position of the endoscope during advancement and/or withdrawal into the patient.

### BRIEF SUMMARY OF THE INVENTION

The information on the length of an endoscope or colonoscope inserted into a body organ within a patient may be used to aid in mapping the body organ, anatomical landmarks, anomalies, etc., and/or to maintain real-time knowledge along the entire length of the endoscope position within the body. This is particularly useful when used in conjunction with various endoscopes and/or colonoscopes having a distal steerable portion and an automatically controlled proximal portion which may be automatically controlled by, e.g., a controller. Examples of such devices are described in detail in the following granted patents and co-pending applications: U.S. Pat. No. 6,468,203; U.S. Pat. App. No. 09/969,927 filed October 2, 2001; U.S. Pat. App. No. 10/229,577 filed August 27, 2002; U.S. Pat. App. No. 10/087,100 filed March 1, 2002; and U.S. Pat. App. No. 10/139,289 filed May 2, 2002.

One method, which is not part of the invention, for determining endoscopic insertion depth and/or position is to utilize a fully instrumented endoscopic device which incorporates features or elements configured to determine the endoscope's depth of insertion without the need for a separate or external sensing device and to relay this information to the operator, surgeon, nurse, or technician involved in carrying out a procedure. Another method, which is not part of the invention, is to utilize a sensing device separate from and external to the endoscope that may or may not be connected to the endoscope and which interacts with the endoscope to determine which portion of the endoscope has passed through or by a reference boundary. The external sensing device may also be referred to herein interchangeably as a datum or datum device as it may function, in part, as a point of reference relative to a position of the endoscope and/or patient. This datum may be located externally of the endoscope and either internally or externally to the body of the patient; thus, the interaction between the endoscope and the datum may be through direct contact or through non-contact interactions.

An instrumented endoscope may accomplish measurement by polling the status of the entire scope (or at least a portion of the scope length), and then determining the endoscope position in relation to an anatomical boundary or landmark such as, e.g., the anus in the case of a colonoscope. The polled information may be obtained by a number of sensors located along the length of the device. Because the sensed information may be obtained from the entire endoscope length (or at least a portion of its length), the direction of endoscope insertion or withdrawal from the body may be omitted because the instantaneous status of the endoscope may be provided by the sensors.

Aside from endoscopes being instrumented to measure insertion depth, other endoscope variations may be used in conjunction with a separate and external device that may or may not be attached to the body and which is configured to measure and/or record endoscope insertion depth. This device may be referred to as an external sensing device or as a datum or datum device. These terms are used interchangeably herein as the external sensing device may function, in part, as a point of reference relative to a position of the endoscope and/or patient. This datum may be located externally of the endoscope and either internally or externally of the body of the patient; thus, the interaction between the endoscope and the datum may be through direct contact or through non-contact interactions. Moreover, the datum may be configured to sense or read positional information by polling the status of sensors, which may be located along the body of the endoscope, as the endoscope passes into the body through, e.g., the anus. The datum may be positioned external to the patient and located, e.g., on the bed or platform that the patient is positioned upon, attached to a separate cart, or removably attached to the patient body, etc.

If the patient is positioned so that they are unable to move with any significant movement during a procedure, the datum may function as a fixed point of reference by securing it to another fixed point in the room. Alternatively, the datum may be attached directly to the patient in a fixed location relative to the point of entry of the endoscope into the patient's body. For instance, for colonoscopic procedures the datum may be positioned on the patient's body near the anus. The location where the datum is positioned is ideally a place that moves minimally relative to the anus because during such a procedure, the patient may shift position, twitch, flex, etc., and disturb the measurement of the endoscope. Therefore, the datum may be positioned in one of several places on the body.

One location may be along the natal cleft, i.e., the crease defined between the gluteal muscles typically extending from the anus towards the lower back. The natal cleft generally has little or no fat layers or musculature and does not move appreciably relative to.the anus. Another location may be directly on the gluteal muscle adjacent to the anus.

### BRIEF DESCRIPTION OF THE DRAWINGS

Fig. 1A shows an example of an endoscope having an electrical circuit throughout the length of the instrument.

Fig. 1B shows an example of the device of Fig. 1A prior to being inserted into a patient.

Fig. 1C shows a device sensing its position as it is advanced through the anus of the patient.

Fig. 1D shows a cross-sectional view of one variation of the endoscope of Fig. 1A.

Figs. 2A and 2B show an endoscopic device having a series of individual sensors or switches for sensing its insertion depth or position.

Fig. 3A shows another example of an endoscope which may have a number of sensors positioned along the length at discrete locations.

Fig. 3B shows the device of Fig. 3A with individual sensor wires leading to each of the sensors along the length.

Fig. 4 shows another example in which pairs of sensor wires may be placed along the length of the endoscope terminating at discrete locations.

Figs. 5A to 5D show another example of an endoscope in which the endoscope position may be determined in part by the resistance measured between adjacent sensor rings.

Fig. 6 shows an example of an algorithm which may be utilized for determining and recording insertion depth of an endoscope.

Figs. 7A and 7B show an example of an endoscope which may utilize an external device for determining endoscope position.

Fig. 7C shows another example of an endoscope having a non-uniform diameter utilizing an external device for determining endoscope position.

Fig. 8 shows another example of an external device which may be used to determine endoscope position.

Fig. 9 shows another example of an external device which may be used to detect sensors positioned on the endoscope.

Fig. 10 shows one example of determining endoscope insertion and/or withdrawal using at least two sensors.

Figs. 11A and 11B show examples of plots indicating sensor readings from the two sensors of Fig. 10 which may be used to determine whether the endoscope is being advanced or withdrawn.

Figs. 12A to 12D show at least four situations, respectively, on how the direction of travel for the endoscope may be determined using the two sensors of Fig. 10.

Fig. 13 shows an example of an algorithm which may be utilized for determining the endoscope direction of travel.

Fig. 14 shows a simplified example for determining endoscope position with an external device.

Fig. 15 shows an example illustrating the positioning which may be utilized for an external device with an endoscope.

Fig. 16 shows a schematic variation utilizing a single magnetic device and multiple sensors.

Figs. 17A and 17B illustrate one example for sensing individual segments of an endoscopic device as it passes the sensor.

Fig. 18 shows another example for sensing individual segments of an endoscopic device having discrete permanent magnets or electromagnets positioned along the endoscope.

Figs. 19A and 19B illustrate another example for sensing individual segments of an endoscopic device using multiple permanent magnets or electromagnets.

Fig. 20 shows only the vertebrae of an endoscopic device, for clarity, with discrete permanent magnets or electromagnets positioned along the endoscope.

Figs. 21A and 21B show side and cross-sectional views, respectively, of another example for magnet positioning along the endoscope.

Figs. 22A and 22B show another example for applying ferrous material, other materials that may alter or affect a magnetic field, permanent magnets, or electromagnets along the endoscope.

Fig. 23 shows another example in which magnets or ferrous material, or other materials that may alter or affect a magnetic field, may be positioned along an elongate support or tool which may then be positioned within the working lumen of a conventional endoscope.

Figs. 24A to 24C show various examples for attaching ferrous materials or other materials that may alter or affect a magnetic field to individual vertebrae of an endoscope.

Figs. 25A and 25B show examples of alternative sensing mechanisms using, e.g., force measurement.

Figs. 26A and 26B show another example of alternative sensing mechanisms using, e.g., a rotatable wheel having discrete permanent magnets or electromagnets integrated within or upon the wheel.

Fig. 27 shows one example of a datum which may be positioned along or within the natal cleft.

Fig. 28 shows another example of a datum which may also be aligned along or within the natal cleft using a flexible and elongate member.

Figs. 29A and 29B show one possible configuration for the.datum sensor.

Figs. 30A and 30B show another example of datum positioning for securing the sensor to the patient

Fig. 31 shows another example of a datum for use with a sensor within a disposable substrate.

Figs. 32A and 32B show another example of a datum which may be positioned on a single cheek adjacent to the anus.

Figs. 33A to 33C show another example of a datum which may also be positioned on a single cheek adjacent to the anus.

Fig. 34 shows yet another example of a datum which may also be positioned on a single cheek adjacent to the anus.

Fig. 35 shows yet another example of a datum having multiple sensors which may also be positioned on a single cheek adjacent to the anus.

Fig. 36 shows an example of an encased datum.

Fig. 37 shows an example of a datum which may be placed upon both cheeks while spanning the natal cleft.

Figs. 38A and 38B show an example of a datum which may be used to encircle the endoscope when in use.

Fig. 39 shows an example of a datum which may be incorporated into the fabric of an undergarment in the region surrounding the anus.

### DETAILED DESCRIPTION OF THE INVENTION

A determination of the length of an endoscope or colonoscope inserted into a body organ within a patient, or generally into any enclosed space, is useful information which may be used to aid in mapping the body organ, anatomical landmarks, anomalies, etc., and/or to maintain real-time knowledge of the endoscope position within the body. The term endoscope and colonoscope may be used herein interchangeably but shall refer to the same type of device. This is particularly useful when used in conjunction with various endoscopes and/or colonoscopes having a distal steerable portion and an automatically controlled proximal portion which may be automatically controlled by, e.g., a controller. Examples of such devices are described in detail in the following granted patents and co-pending applications: U.S. Pat. No. 6,468,203; U.S. Pat. App. No. 09/969,927 filed October 2,2001; U.S. Pat. App. No. 10/229,577 filed August 27, 2002; U.S. Pat. App. No. 10/087,100 filed March 1, 2002; and U.S. Pat. App. No. 10/139,289 filed May 2, 2002.

There are at least two different approaches which may be utilized in determining endoscopic insertion depth and/or position when an endoscope has been inserted within the body. One method, which is not part of the invention, is to utilize a fully instrumented endoscopic device which incorporates features or elements which are configured to determine the endoscope's depth of insertion and to relay this information to the operator, surgeon, nurse, or technician involved in carrying out a procedure.

Another method, which is not part of the invention, is to utilize a sensing device separate from and external to the endoscope and which interacts with the endoscope to determine which portion of the endoscope has passed through or by a reference boundary. The external sensing device may also be referred to herein interchangeably as a datum or datum device as it may function, in part, as a point of reference relative to a position of the endoscope and/or patient. This datum may be located externally of the endoscope and either internally or externally to the body of the patient; thus, the interaction between the endoscope and the datum may be through direct contact or through non-contact interactions.

### INSTRUMENTED ENDOSCOPES

One method of determination for endoscopic insertion depth and/or position, which is not part of the invention, is through an endoscopic device which may be configured to determine its depth of insertion. That is, an endoscopic device may be configured to indicate the portion of the endoscope that has been inserted into a body organ without the need for a separate or external sensing device. This type of determination may reflect an endoscope configured such that its depth measurement is independent of its progress during insertion or withdrawal into the body organ and instead reflects its depth instantaneously without regards to its insertion history.

Such an endoscopic device may accomplish this, in part, by polling the status of the entire scope (or at least a portion of the scope length), and then determining the endoscope position in relation to an anatomical boundary or landmark such as, e.g., the anus in the case of a colonoscope. The polled information may be obtained by a number of sensors located along the length of the device, as described in further detail below. Because the sensed information may be obtained from the entire endoscope length (or at least a portion of its length), the direction of endoscope insertion or withdrawal from the body may be omitted because the instantaneous status of the endoscope may be provided by the sensors. Directional information or history of the endoscope position during an exploratory or diagnostic procedure may optionally be recorded and/or stored by reviewing the endoscope time history of insertion depth.

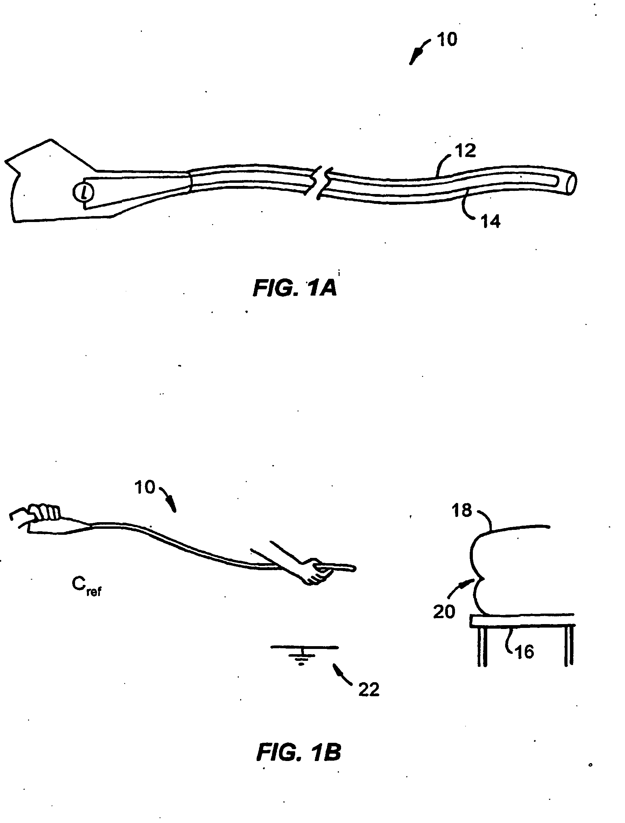

One variation is seen in Fig. 1A which shows endoscope assembly **10.** Endoscope **12** may be configured to have at least a single circuit **14** wired through the length of the shaft of endoscope **12.** Circuit **14** may also be wired through only a portion of the shaft length or through a majority of the shaft length depending upon the desired proportion of the shaft that the operator, surgeon, or technician desires to act as a sensor. The single circuit **14** may thus configure the endoscope **12** to function as a single continuous sensor. Depending upon the type of sensors implemented, as described in further detail below, changes in an output variable received by the sensors may be measured and recorded. The degree of change in the output variable may then be correlated to the length of the endoscope **12** inserted into the body. The change in the output variable may also be based upon varying environmental factors experienced by the endoscope **12.** For instance, one example of an environmental factor which may instigate changes in the output variable sensed by the circuit **14** may include pressure sensed from the surrounding tissue, e.g., from the anus, where endoscope **12** is initially inserted into the body. Another factor may include changes in electrical conductivity, e.g., from the tissue, when the endoscope **12** is inserted into the body.

Endoscope **12** may alternatively be configured to detect and correlate the length of the endoscope **12** remaining outside the body rather than inside the body to indirectly calculate the insertion depth. Moreover, the endoscope **12** may additionally detect and correlate both the length of the endoscope **12** remaining outside the body as well as the length of endoscope **12** inserted within the body. Alternatively, endoscope **12** may sense the location of the orifice or anus 20 along the length of the device and then calculate either the length remaining outside the body or the insertion length relative to the position of anus **20.**

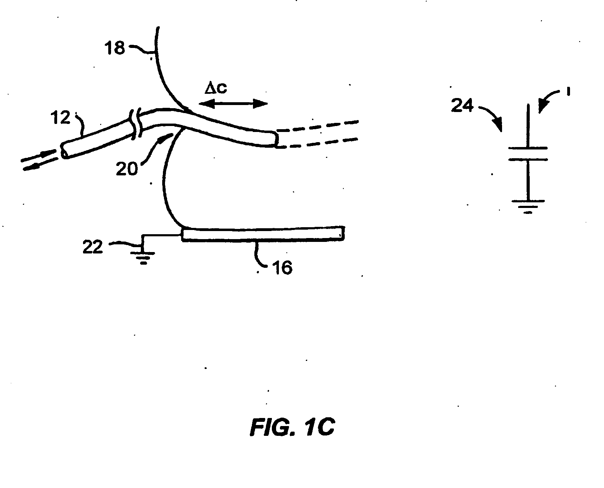

Another example of changing environmental factors leading to a change in an output variable is shown in Figs. **1B** and 1C, which show an example of endoscope assembly **10** configured as a capacitive sensing endoscopic device. As seen in Fig. 1B, patient **18** may be positioned upon table and/or grounding pad **16** which may be connected to electrical ground **22.** Fig. 1C shows endoscope **12** inserted within anus **20** of patient **18.** Prior to or while endoscope **12** is inserted in patient **18,** a constant input current may be provided to endoscope **12** and the voltage may be measured in accordance. Endoscope **12** may thus act as a plate within a capacitor while grounding pad **16** placed under patient **18** may function as a second opposing plate to endoscope **12,** as represented in the schematic **24.** The resulting capacitance between endoscope **12** and grounding pad **16** may be calculated based upon the value of the current, i, over a time period, t, and/or upon the measured difference in phase shift between the input frequency and the resulting frequency. As endoscope **12** is inserted or withdrawn from anus **20,** the calculated capacitance will vary according to differences in the dielectric constants between the tissue of patient **18** and that of air. This capacitance change may be constantly monitored and mapped against the length of endoscope **12** to indicate the length of insertion within patient **18.**

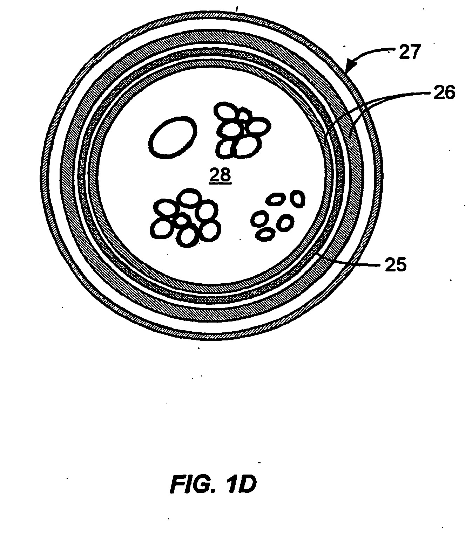

Another variation on endoscopic sensing may utilize resistivity rather than capacitance. For instance, continuous circuit **14** may be configured into a single printed circuit with an overlay of conductive printed carbon. Fig. 1D shows one variation on a cross-section of endoscope **12** which may be configured as such. As seen, conductive printed carbon layer **25** may be positioned circumferentially within printed flex circuit **26** while surrounding endoscope interior **28.** The endoscope **12** may be optionally covered by an outer jacket or sheath **27** to cover the endoscope and its electronics. In use, when the endoscope **12** is inserted into the patient **18** through, e.g., the anus **20,** pressure from the surrounding tissue at the point of insertion into the body may force contact between carbon layer **25** and flex circuit **26** within endoscope **12** and thereby close the circuit **14** at the point of insertion. As endoscope **12** is inserted and withdrawn from anus **20,** the contact point between carbon layer **25** and flex circuit **26** will vary according to where the pressure is applied at the point of insertion and the resistance of the circuit **14** at any one time may be measured and mapped against the length of endoscope **12** to indicate the length of insertion within anus **20.**

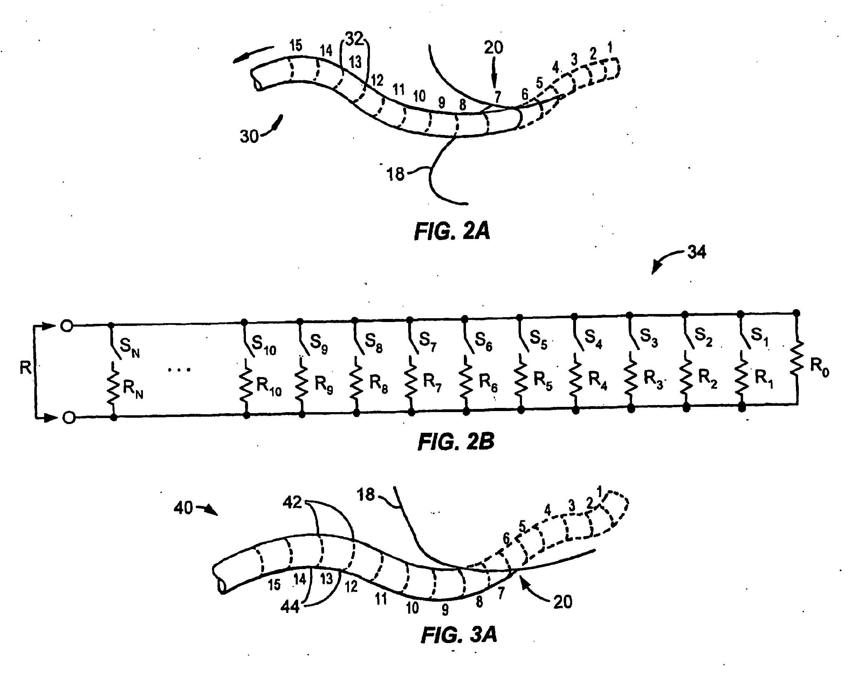

Another variation is shown in Figs. **2A** and **2B****,** which show an endoscopic device having a series of individual sensors or switches for sensing its insertion depth or position. Endoscope **30** is shown as having a continuous circuit with a plurality of open, individual switches or conductive sections **32** positioned along the length of the device **30.** Switches, S_{I} to S_{N}, may be positioned at regular intervals along endoscope **12.** The spacing between the switches may vary and may depend upon the desired degree of accuracy in endoscope position determination. Switches may be positioned closely to one another to provide for a more accurate reading, while switches spaced farther apart from one another may provide for a less accurate determination. Moreover, the switches may be positioned at uniform distances from one another, or alternatively they may be spaced apart at irregular intervals, depending upon the desired results. The switches may also take a variety of electrically conductive forms, e.g., membrane switches, force sensitive resistors (FSR), etc.

Another variation on the type of switch which may be used is light-detecting transducers. The switches S₁ to S_{N}, may be configured as one of a variety of different types of photo-sensitive switches, e.g., photoemissive detectors, photoconductive cells, photovoltaic cells, photodiodes, phototransistors, etc. The switches S₁ to S_{N}, may be located at predetermined positions along the length of the endoscope **30.** As the endoscope **30** is inserted into the patient **18,** the change in ambient light from outside the patient **18** to inside the patient **18** may result in a voltage change in the switches inserted within the body **18..** This transition may thereby indicate the insertion depth of the endoscope **30** within the body **18** or the length of the endoscope **30** still located outside the body **18.** The types of photo-sensitive switches aforementioned may have a current running through them during a procedure, with the exception of photovoltaic switches, which may be powered entirely by the ambient light outside the body **18.**

Fig. 2B shows a schematic representation **34** of the device of Fig. 2A. As shown, switches, S₁ to S_{N}, may be configured such that they are in parallel to one another. Insertion or withdrawal of the endoscope **12** within patient **18** may activate or close a switch through, e.g., interaction with electrically conductive tissue, pressure from the anus closing the switch, changes in moisture or pH, temperature changes, light intensity changes, etc. The closing of a particular switch will vary according to how deep the endoscope **12** is inserted within the anus **20.** When a particular switch is electrically activated, a corresponding resistance value, ranging from R₁ to R_{N}, may be measured and then mapped against the endoscope **12** to indicate the length of insertion.

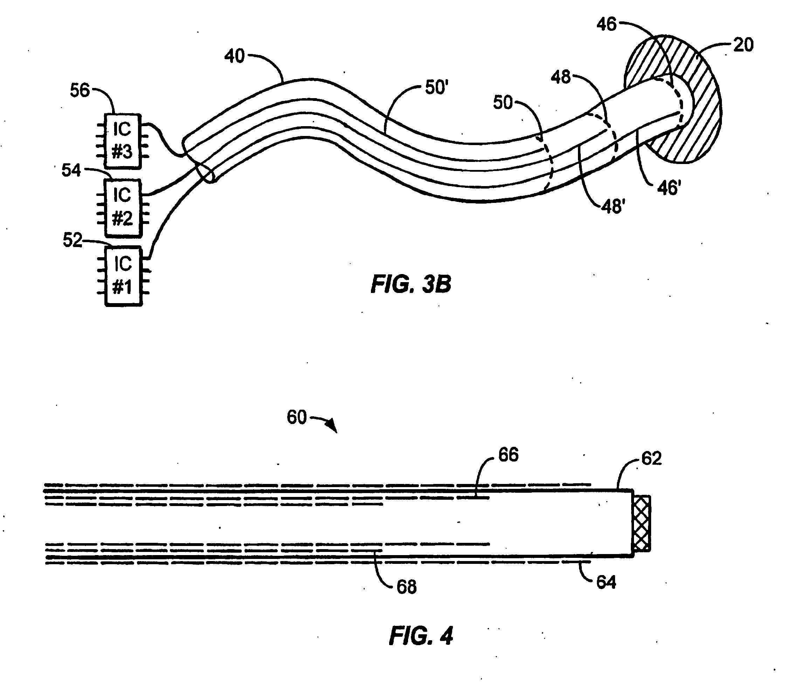

Another variation is shown in Figs. 3A and 3B which show an endoscope **40** having a number of sensors positioned along the length of the endoscope **40** at discrete locations. In this variation, a number of sensor wires may be placed along the length of the endoscope **12** such that each wire terminates at subsequent locations along the endoscope **12,** as shown in Fig. 3B. Although only three wires are shown, this is merely intended to be illustrative and any number of fewer or additional wires may be utilized depending upon the desired length of the endoscope **12** to be instrumented. The placement of the distal ends of sensor wires **46', 48', 50'** may coincide with the number of vertebrae or links of the endoscope **12** structure. The sensor wires **46', 48', 50'** may be simply routed through within the endoscope **12** length or they may be placed along the exterior of the device. The distal ends of the wires may be exposed to allow for communication with the tissue or they may alternatively be each connected to corresponding conductors **42** which divide the endoscope **12** up into a number of segments **44.** These optional conductors **42** may be formed in the shape of rings to allow for circumferential contact with the tissue. Each sensor wire **46', 48', 50'** may thus be in electrical communication with a corresponding conductor **46, 48, 50,** respectively, and so on, depending upon the number of wires and corresponding conductors utilized. The individual sensors may also be networked together on a single bus and more complex networking and placement of sensors may also be implemented to yield additional information, e.g., rotational position of the endoscope **12.** The proximal ends of the sensor wires **46', 48', 50'** may each be connected to a corresponding processor **52, 54, 56,** respectively, such that the length of the endoscope **12** inserted within the anus **20** may be determined by polling the status of each individual sensor wire **46', 48', 50'.**

Fig. 4 shows another endoscopic assembly variation **60** in which corresponding pairs of wire sensors may be positioned along an endoscope **62** body. A first pair 64 of wire sensors may extend along the endoscope **62** and terminate at a first distal location; a second pair **66** of wire sensors may also extend along the endoscope **62** and terminate at a second distal location which is proximal of the first distal location; and a third pair **68** of wire sensors may also extend along the endoscope **62** and terminate at a third distal location which is proximal of the second distal location, and so on. Any number of wire pairs may be used and the distances between each of the first, second, third, etc., distal locations may be uniform or irregular, depending upon the desired measurement results. This variation 60 may operate in the same manner as above by measuring which pair of wire sensors is disrupted when inserted or withdrawn from a patient.

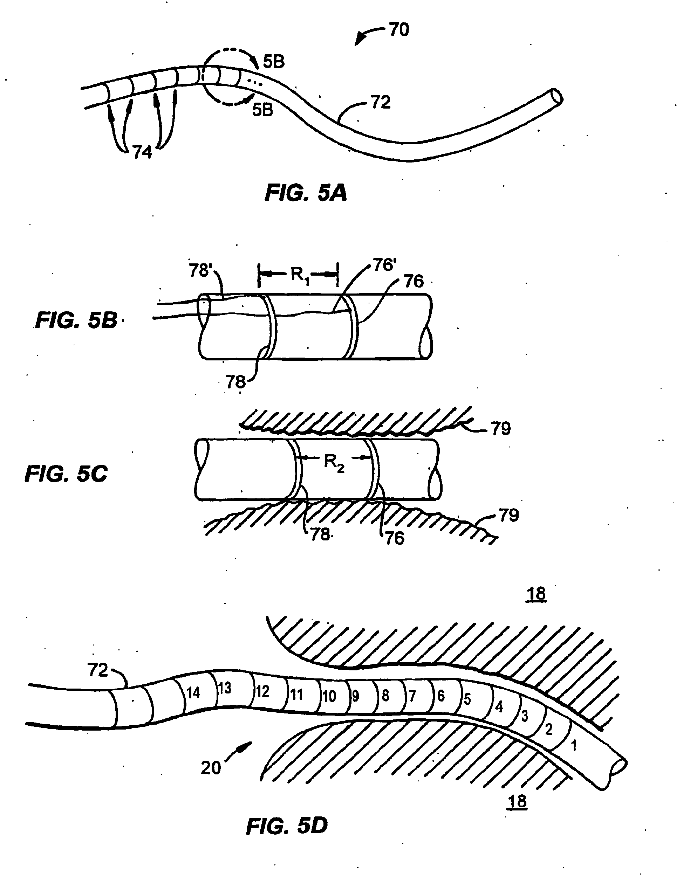

Yet another example is shown in Figs. 5A to 5D which shows endoscope assembly 70 which may comprise an endoscope **72** having at least one or more, preferably at least two or more, conductive sensors **74** positioned along the length of endoscope **72.** Sensors **74** may be in the shape of rings and may be further configured to measure resistance between each adjacent ring. Fig. **5B** is a detailed view of a portion of endoscope **72** which shows first sensor **76** and adjacent second sensor **78.** Each sensor **76, 78** may be connected to a separate sensor wire **76', 78'** such that the electrical resistance, e.g., **R₁,** between adjacent sensors, e.g., sensors **76, 78,** may be measured when contacting a region of tissue. Fig. 5C shows sensors **76, 78** contacting tissue **79.** As the endoscope **72** is advanced or withdrawn from the tissue, resistance values between adjacent sensors may be measured to determine the position of the endoscope **72** within the patient 18. As seen in Fig. 5D, resistance values may be subsequently measured between each adjacent sensor, shown as sensors 1, 2, 3, etc., as the device is advanced into patient **18.** This may be accomplished, in part, by correlating measured resistance values between sensors where R ≈∞when sensors are measured outside of the body, and R << ∞when sensors are measured inside the body when surrounded by tissue.

As mentioned above, other output variables aside from pressure or force, capacitance, and resistance measurements may also be employed to determine endoscopic insertion depth. For instance, moisture or pH sensors may be utilized since moisture or pH values change dramatically with insertion into the body. Temperature or heat flux sensing may also be utilized by placing temperature sensors, e.g., thermistors, thermocouples, etc., at varying locations along the endoscope body. Temperature sensing may take advantage of the temperature differences between air and the body. Another alternative may include heating or cooling the interior of the endoscope at ranges above or below body temperature. Thus, the resultant heat flux into or out of the endoscope, depending upon the interior endoscope temperature, may be monitored to determine which portion of the endoscope are in contact with the body tissue. Another alternative may include light sensing by positioning light sensors at locations along the endoscope body. Thus, light intensity differences may be determined between outside and inside the body to map endoscope insertion depth. Alternatively, sound waves or other pressure waves, ultrasound, inductive proximity sensors, etc., may also be utilized.

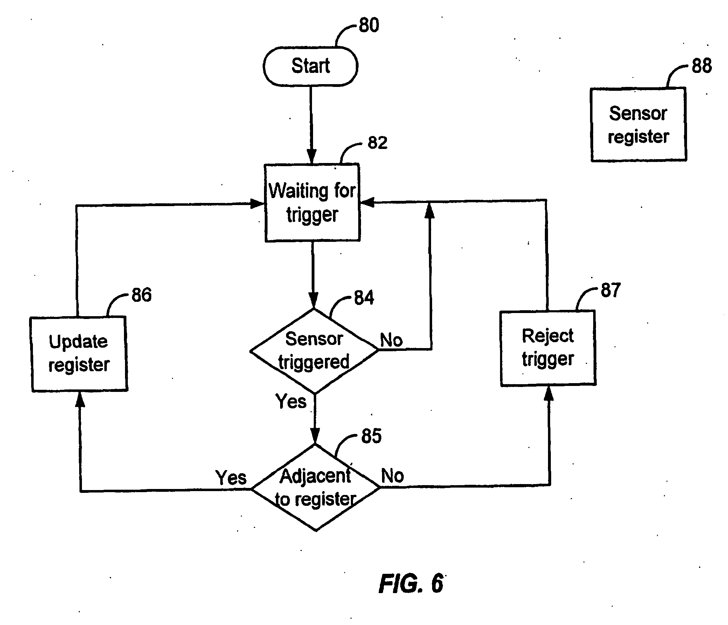

In utilizing sensors positioned upon the endoscope body, an algorithm may be utilized for determining and recording the insertion depth of the endoscope within a patient, as shown in Fig. 6. This variation on an algorithm operates on the general principle that each of the sensors are triggered sequentially as the endoscope is inserted or withdrawn from the patient. A register may be used to record and keep track of the latest insertion depth, i.e., the most recent and valid triggered sensor. The endoscope and algorithm may be configured such that sensor readings that are considered valid are those readings which are triggered by the same sensor or adjacent sensors such that insertion, withdrawal, or no motion may be indicated. Other sensor triggers can be ignored or rejected while valid sensor triggers may cause the register to update.

Such an algorithm may be implemented with any of the devices described above to eliminate false measurements and to maintain accurate insertion depth measurements: Step **80** indicates the start of the algorithm as the endoscope waits for a sensor to be triggered **82.** If a sensor has not been triggered **84,** the algorithm would indicate a "No" and the device would continue to wait for a trigger signal. Upon an indication that a sensor has been triggered **84,** a comparison of the triggered signal takes place to compare whether the sensed signal is from an adjacent sensor **85** by comparing the triggered sensor information to stored register information in sensor register **88.** If the triggered signal is not from an adjacent sensor, the signal is rejected as a false signal **87** and the endoscope goes back to waiting for a sensor to be triggered **82.** However, if the triggered signal is from an adjacent sensor when compared to the value stored in register **88,** register **88** is updated **86** with the new sensor information and the endoscope then continues to wait for another sensor to be triggered **82.**

### ENDOSCOPES USING EXTERNAL SENSING DEVICES

Aside from endoscopes being instrumented to measure insertion depth, other endoscopes may be used in conjunction with a separate device configured to measure and/or record endoscope insertion depth. This separate device may be referred to as an external sensing device or as a datum or datum device. These terms are used interchangeably herein as the external sensing device may function, in part, as a point of reference relative to a position of the endoscope and/or patient. This datum may be located externally of the endoscope and either internally or externally to the body of the patient; thus, the interaction between the endoscope and the datum may be through direct contact or through non-contact interactions. Moreover, the datum may be configured to sense or read positional information by polling the status of sensors or transponders, which may be located along the body of the endoscope, as the endoscope passes into the body through, e.g., the anus. Alternatively, the datum may be configured to detect sensors or transponders only within a limited region or area. The datum may be positioned external to the patient and located, e.g., on the bed or platform that the patient is positioned upon, attached to a separate cart, or removably attached either internally or externally to the patient body, etc.

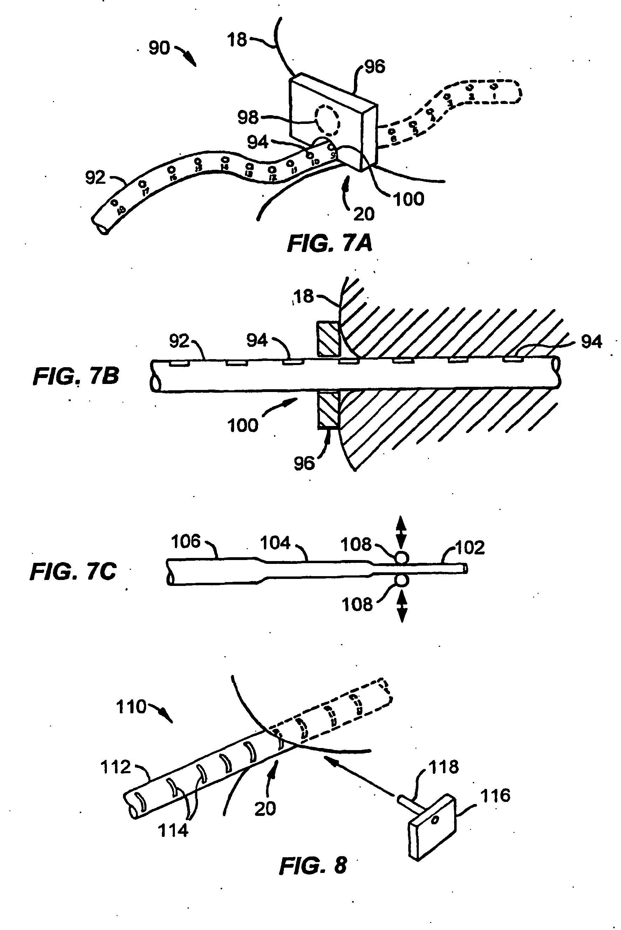

Figs. 7A and 7B show one variation in using an endoscope assembly **90** in conjunction with external sensing device or datum **96.** Datum **96** may be positioned externally of patient **18** adjacent to an opening into a body cavity, e.g., anus **20** for colonoscopic procedures. Datum **96** may accordingly have a sensor or reader **98** located next to opening **100,** which may be used as a guide for passage of endoscope **92** therethrough into anus **20.** Endoscope **92** may be configured to have a number of tags **94,** e.g., sensors, transponders, etc., located along the body of endoscope **92.** These tags **94** may be positioned at regular intervals along endoscope **92.** The spacing between the tags **94** may vary and may also depend upon the desired degree of accuracy in endoscope position determination. Tags **94** may be positioned closely to one another to provide for a more accurate reading, while tags **94** spaced farther apart from one another may provide for a less accurate determination. Moreover, tags **94** may be positioned at uniform distances from one another, or alternatively they may be spaced apart are irregular intervals, depending upon the desired results. Moreover, tags **94** may be positioned along the entire length of endoscope **92** or only along a portion of it, depending upon the desired results. As shown in Fig. **7B****,** as endoscope **92** is passed through datum **96** via opening **100** and into anus **20,** reader **98** located within datum **96** may sense each of the tags **94** as they pass through opening **100.** Accordingly, the direction and insertion depth of endoscope **92** may be recorded and/or maintained for real-time positional information of the endoscope **92.**

Any number of technologies may be utilized with tags **94.** For instance, one variation may have tags **94** configured as RF identification tags or antennas. Reader **98** may accordingly be configured as a RF receiving device. Each tag **94** may be encoded with, e.g., position information such as the distance of a particular tag **94** from the distal end of endoscope **92.** The reader **98** may be configured to thus read in only certain regions or zones, e.g., reader **98** may read only those RF tags passing through opening **100** or only those tags adjacent to anus **20.** Alternatively, the RF tags may be configured to transmit the status of, e.g., pressure switches as described above, to datum **96** to determine the length of insertion.

Another variation on tags **94** may be to configure the tags for ultrasonic sensing. For example, each tag **94** may be configured as piezoelectric transducers or speakers positioned along the endoscope **92.** The reader **98** may thus be configured as an ultrasonic receiver for receiving positional information from tuned transducers or tags **94** each of which relay its positional information. Alternatively, optical sensors may be used as tags **94.** In this variation, each tag **94** may be configured as a passive encoded marker located on an outer surface of endoscope **92.** These markers may be in the form of a conventional bar code, custom bar code, color patterns, etc., and each may be further configured to indicate directional motion, i.e., insertion or withdrawal. Furthermore, each tag **94** may be configured as active encoded markers, e.g., LEDs which may be blinking in coded patterns. Reader **98** may thus be configured as an optical sensor.

Another alternative may be to configure tags **94** and reader **98** for infrared (IR) sensing in which case IR emitters may be positioned along the length of endoscope **92** such that each IR emitter or tag **94** is configured to emit light at a specific frequency according to its position along the endoscope **92.** Reader **98** may thus be configured as an IR receiver for receiving the different frequencies of light and mapping the specific frequency detected against the length of endoscope **92.** Yet another alternative may be to have tags **94** configured magnetically such that a magnetic reader in datum **96** can read the position of the device, as described in further detail below.

Yet another alternative may be to configure the datum and endoscope assembly as a linear cable transducer assembly. In this variation, reader **98** may be configured as a transducer having a cable, wire, or some other flexible member extending from reader **98** and attached to the distal end of endoscope **92.** While the datum **96** remains external to the patient and further remains in a fixed position relative to the patient, the endoscope **92** may be advanced within the patient while pulling the cable or wire from reader **98.** The proximal end of the cable or wire may be attached to a spool of cable or wire in electrical communication with a multi-tum potentiometer. To retract the cable or wire when the endoscope **92** is withdrawn, the spool may be biased to urge the retraction of the cable or wire back onto the spool. Thus, the change of wire length may be correlated to an output of the reader **98** or of the potentiometer to a length of the extended cable and thus the length of the endoscope **92** inserted within the patient

Yet another alternative may be to mount rollers connected to, e.g., multi-turn potentiometers, encoders, etc., on datum **96.** These rollers may be configured to be in direct contact with the endoscope **92** such that the rollers rotate in a first direction when endoscope **92** is advanced and the rollers rotate in the opposite direction when endoscope **92** is withdrawn. The turning and number of revolutions turned by the rollers may be correlated into a length of the insertion depth of endoscope **92.**

Yet another alternative may be to use the endoscopes, or any of the endoscopes described herein, in conjunction with conventional imaging technologies which are able to produce images within the body of a patient. For instance, any one of the imaging technologies such as x-ray, fluoroscopy, computed tomography (CT), magnetic resonance imaging (MRI), magnetic field location systems, etc., may be used in conjunction with the endoscopes described herein for determining the insertion depth.

In yet another alternative, the datum may be used to sense the positional information from the endoscope through the use of one or several pressure sensors located on the datum, e.g., datum **96.** The pressure sensor may be positioned upon datum **96** such that it may press up against the endoscope **92** as it is advanced or withdrawn. This pressure sensor may be configured, e.g., as a switch, or it alternatively be configured to sense certain features on the endoscope **92,** e.g., patterned textures, depressions, detents, etc., which are located at predetermined lengths or length intervals to indicate to the pressure switch the insertion depth of endoscope **92.**

Yet another alternative is to sense changes in the diameter of the endoscope body inserted into the patient, as seen in Fig. 7C. The insertion length of the endoscope may have multiple sections each having a unique diameter, e.g., a distal most section **102** may have the smallest diameter and each successive proximal section **104,106** may have incrementally larger diameters. Alternatively, successive sections may have alternating diameter sizes where a first section may have a first diameter, a second section may have a second larger diameter, and the third section may have a diameter equal to the first diameter or larger than the second diameter, and so on. The differences in endoscopic diameter may be used to detect the endoscopic insertion depth by using a datum 108 which may be configured to maintain contact with the endoscope and move according to the diameter changes of the endoscope, as shown by the arrows. This diameter referencing device and method, which is not part of the invention, may be used independently or in conjunction with any of the other methods described herein as a check to ensure that the position of the endoscope concurs with the results using other methods of sensing.

Fig. 8 shows another example in endoscope assembly **110** in which endoscope **112** may have a number of sensors or tags **114** located along the body of the endoscope **112.** As endoscope **112** is advanced or withdrawn from anus **20,** datum **116,** which may be mounted externally of the patient and at a distance from endoscope **112,** may have a receiver or reader **118** configured in any of the variations described above. For instance, receiver or reader **118** may be adapted to function as a RF receiver, ultrasonic receiver, optical sensor, or as any of the other variations described above, to read only those tags **114** adjacent to anus **20** and to map their position on the endoscope **112** and thus, the length of insertion.

If reader **118** were configured as an optical sensor, it may further utilize a light source, e.g., LED, laser, carbon, etc., within datum **116.** This light source may be utilized along with a CCD or CMOS imaging system connected to a digital signal processor (DSP) within reader **118.** The light may be used to illuminate markings located at predetermined intervals along endoscope **112.** Alternatively, the markings may be omitted entirely and the CCD or CMOS imaging system may be used to simply detect irregularities normally present along the surface of an endoscope. While the endoscope is moved past the light source and reader 118, the movement of the endoscope may be detected and correlated accordingly to indicate insertion depth.

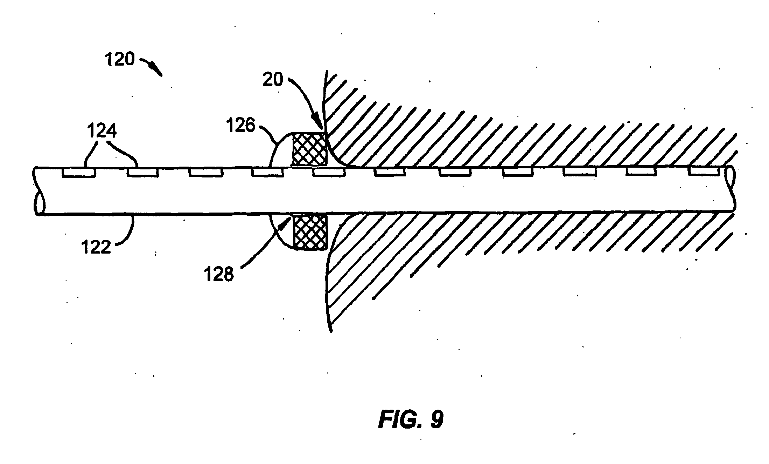

Fig. 9 shows another variation with endoscope assembly **120** in which endoscope **122** may have a number of sensors **124** located along the length of endoscope **122.** These sensors **124** maybe configured as Hall-effect type sensors, as will be described in greater detail below. The datum **126** may be configured as a ring magnet defining an endoscope guide **128** therethrough such that the magnetic field is perpendicularly defined relative to the sensors **124.** Thus, sensors **124** may interact with magnet **126** as they each pass through guide **128.** As a Hall sensor **124** passes through datum **126,** the sensor **124** may experience a voltage difference indicating the passage of a certain sensor through datum 126. These types of sensors will be described in greater detail below.

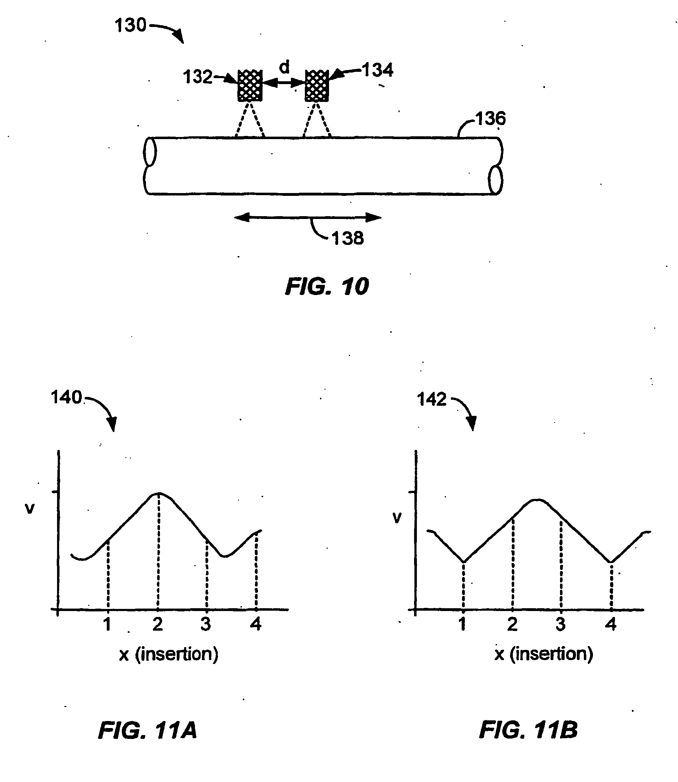

In order to determine the direction of the endoscope when it is either advanced or withdrawn from the patient, directional information may be obtained using any of the examples described above. Another example is to utilize at least two or more sensors positioned at a predetermined distance from one another. Fig. 10 shows one variation illustrating sensor detection assembly **130** with first sensor 132 and second sensor **134.** First and second sensors **132, 134** may be positioned at a predetermined distance, d, from one another. As endoscope **136** is advanced or withdrawn past sensor assembly **130,** the direction of travel **138** of endoscope **136** may be determined by examining and comparing the signals received from each sensor **132,134.** By determining which sensor has a rising edge or input signal first received relative to the other sensor, the direction of travel **138** may be determined. As shown in Fig. 11A, plot **140** generally illustrates signals received from first sensor **132.** From position x =1 to position x = 2, a rise in the signal is measured thus sensing a peak in advance of the signal measured from position x =1 to position x = 2 in plot **142,** which is the signal received from second sensor **134,** as seen in Fig. 11B. Thus, a first direction of travel, e.g., insertion, may be indicated by the relative comparisons between signals in plots **140** and **142.** If endoscope **136** were traveling in the opposite direction, e.g., withdrawal, second sensor **134** would sense a peak in advance of first sensor **132.**

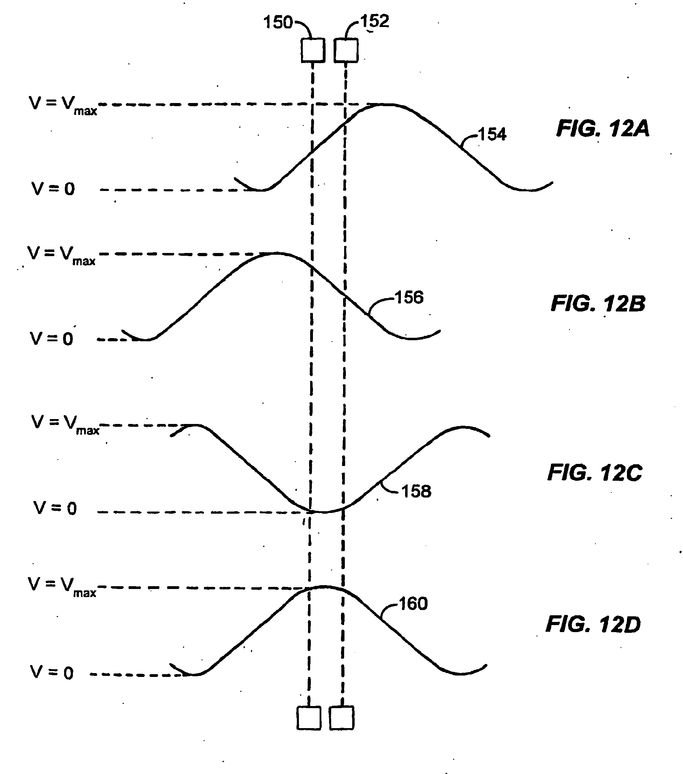

A more detailed description for determining the endoscope's direction of travel follows below. Figs. 12A to 12D illustrate various cases for determining endoscopic direction of travel using first sensor **150** and second sensor 152. First and second sensors **150,152** are preferably at a predetermined distance from one another while an endoscope is passed adjacent to the sensors. For the purposes of this illustration, a direction to the right shall indicate a first direction of travel for an endoscope device, e.g., insertion into a body, while a direction to the left shall indicate a second direction of travel opposite to the first direction, e.g., withdrawal from the body.

Fig. 12A shows a situation in which first sensor **150** measures a voltage less than the voltage measured by second sensor **152,** as indicated by plot **154.** If first and second sensors **150,152** both measure a decrease in voltage, this may indicate a motion of the endoscope to the right while an increase voltage in both first and second sensors **150, 152** may indicate a motion of the endoscope to the left. Fig. 12B shows another situation in which first sensor **150** measures a voltage greater than the voltage measured by second sensor **152,** as indicated by plot **156.** If first and second sensors **150,152** both measure an increase in voltage, this may indicate a motion of the endoscope to the right. However, if both first and second sensors **150,152** measure a decrease in voltage, this may indicate a motion of the endoscope to the left.

Fig. 12C shows another situation where first sensor **150** measures a voltage equal to a voltage measured by second sensor **152,** as shown by plot **158.** In this case, if first sensor **150** measures an increase in voltage prior to second sensor **152** also measuring an increase in voltage, this may be an indication of the endoscope moving to the right. On the other hand, if second sensor **152** measures an increase prior to first sensor **150** measuring an increase in voltage, this may indicate movement of the endoscope to the left. Fig. 12D shows a final situation in plot **160** where first sensor **150** again measures a voltage equal to a voltage measured by second sensor **152.** In this case, the opposite to that shown in Fig. 12C occurs. For instance, if the voltage measured by first sensor **150** decreases prior to the voltage measured by second sensor **152,** this indicates a movement of the endoscope to the right. However, if second sensor **152** measures a voltage which decreases prior to a decrease in voltage measured by first sensor **150,** this may indicate a movement of the endoscope to the left.

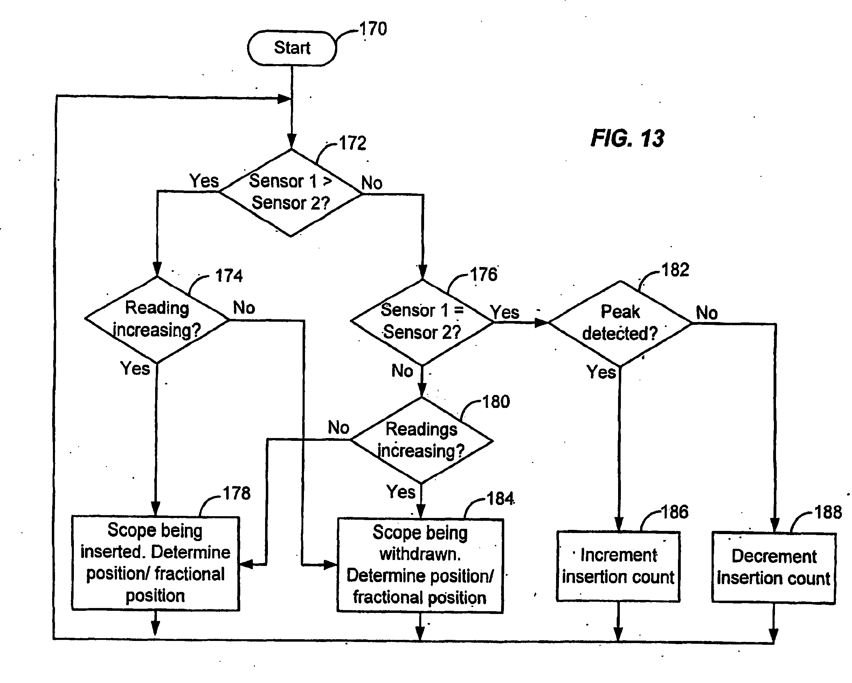

Fig. 13 shows one variation of an algorithm which may be implemented as one method, which is not part of the invention, for determining whether an endoscope is being advanced or withdrawn from the body. Fig. 13 illustrates how the various determinations described above may be combined into one variation for an algorithm. As seen, the algorithm begins with step **170.** In step **172** an initial step of determining whether first sensor **150** measures a voltage greater than second sensor **152** is performed. If first sensor **150** does measure a voltage greater than second sensor **152,** then a second determination may be performed in step **174** where a determination may be made as to whether the voltages measured by both sensors **150, 152** are increasing or not. If both voltages are increasing, step **178** may indicate that the endoscope is being inserted. At this point, the position of the endoscope and its fractional position, i.e., the distance traveled by the endoscope since its last measurement, may be determined and the algorithm may then return to step **172** to await the next measurement.

If, however, first sensor **150** does not measure a voltage greater than second sensor **152** in step **172,** another determination may be performed in step **176 to** determine whether the voltages measured by sensors **150,152** are equal. If the voltages are not equivalent, the algorithm proceeds to step **180 where** yet another determination may be performed in step **180** to determine if both voltages are increasing. If they are not, then step **178** is performed, as described above. If both voltages are increasing, then step **184** may indicate that the endoscope is being withdrawn. At this point, the position of the endoscope and its fractional position, i.e., the distance traveled by the endoscope since its last measurement, may again be determined and the algorithm may then return to step 172 to await the next measurement.

In step **176,** if the voltages measured by first sensor **150** and second sensor **152** are equivalent, then the algorithm may await to determine whether a peak voltage is detected in step **182.** If a peak voltage is detected, step **186** increments the insertion count. However, if a peak is not detected, then step **188** decrements the insertion count. Regardless of whether the insertion count is incremented or decremented, the algorithm may return to step 172 to await the next measurement.

### ENDOSCOPES USING MAGNETIC SENSING DEVICES

One particular variation on measuring endoscopic insertion depth may utilize magnetic sensing, in particular, taking advantage of the Hall effect. Generally, the Hall effect is the appearance of a transverse voltage difference in a sensor, e.g., a conductor, carrying a current perpendicular to a magnetic field. This voltage difference is directly proportional to the flux density through the sensing element. A permanent magnet, electromagnet, or other magnetic field source may be incorporated into a Hall effect sensor to provide the magnetic field. If a passing object, such as another permanent magnet, ferrous material, or other magnetic field-altering material, alters the magnetic field, the change in the Hall-effect voltage may be measured by the transducer.

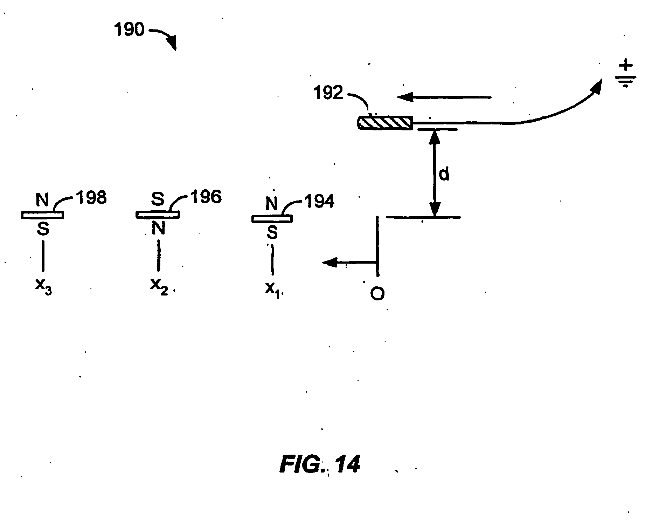

Fig. 14 illustrates generally Hall effect sensor assembly **190** which shows conductor or sensor **192** maintained at a distance, d, as it is passed over magnets **194,196,198** at distances x₁, x₂, x₃, respectively. Each magnet may be positioned such that the polarity df adjacent magnets is opposite to one another or such that the polarity of adjacent magnets is the same. As sensor **192** is passed, voltage differences may be measured to indicate which magnet sensor **192** is adjacent to.

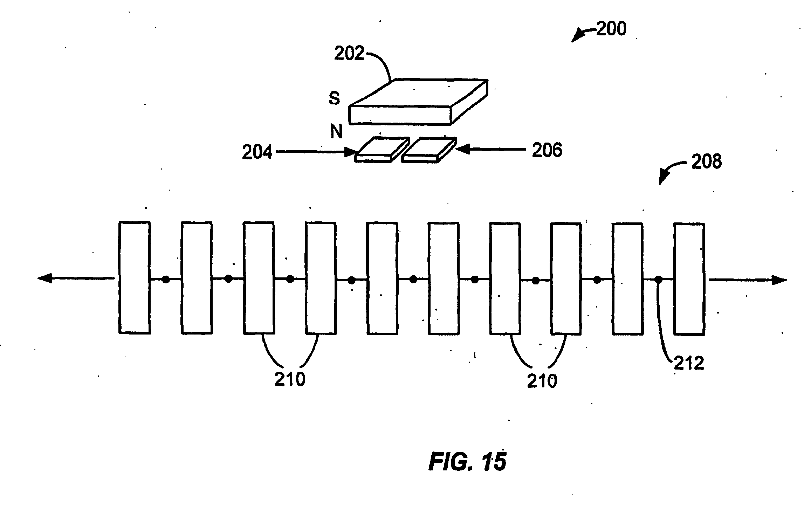

Fig. 15 shows one variation illustrating the general application for implementing Hall effect sensors for endoscopic position measurement. As shown, sensor assembly **200** illustrates one variation having magnet **202** with first sensor **204** and second sensor **206** adjacent to magnet **202.** Magnet **202** may be a permanent magnet or it may also be an electromagnet. First and second sensors **204, 206** are connected to a power supply (not shown) and are positioned from one another at a predetermined distance. Both sensors **204, 206** may also be located at a predetermined distance from magnet **202.** A general representation of endoscope **208** is shown to reveal the individual links or vertebrae **210** that may comprise part of the structure of the endoscope, as described in further detail in any of the references incorporated above. Each vertebrae **210** is shown as being schematically connected to adjacent vertebrae via joints **212** which may allow for endoscope articulation through tortuous paths. Endoscope **208** may be passed by sensor assembly **200** at a predetermined distance as it is inserted or withdrawn from an opening in a patient. Each or a selected number of vertebrae **210** may be made of a ferrous material or other material that may alter or affect a magnetic field or have ferrous materials incorporated in the vertebrae **210.** Thus, as endoscope **208** passes first and second sensors **204, 206,** the ferrous vertebrae **210** may pass through and disrupt a magnetic field generated by magnet **202** and cause a corresponding voltage measurement to be sensed by sensors **204, 206.** Direction of travel for endoscope **208,** i.e., insertion or withdrawal, as well as depth of endoscope insertion may be determined by applying any of the methods, which are not part of the invention, described above.

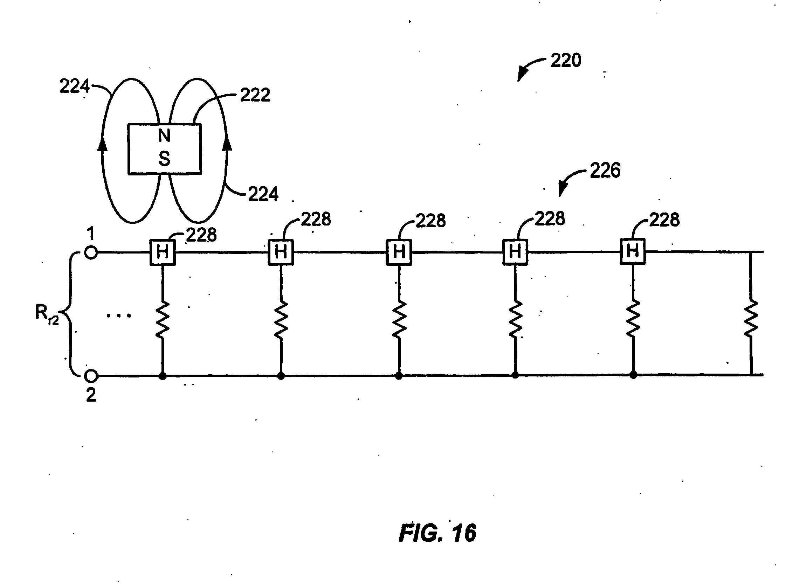

Another variation is shown in Fig. 16 which illustrates a schematic representation **220** of Hall effect sensing in which the sensors may be located on the endoscope **226** itself. Magnet **222** may be positioned adjacent to, e.g., the anus of a patient, such that endoscope **226** passes adjacent to magnet **222** when. inserted or withdrawn from the patient. Endoscope **226** may have a number of discrete Hall switches **228** positioned along the body of endoscope **226.** As endoscope **226** passes magnet **222,** the magnetic field lines **224** may disrupt a switch **228** passing adjacently. Hall switches **228** may be bipolar, unipolar, latched, analog, etc. and may be used to determine the total resistance R₁₋₂ in order to determine insertion length of the endoscope **226.**

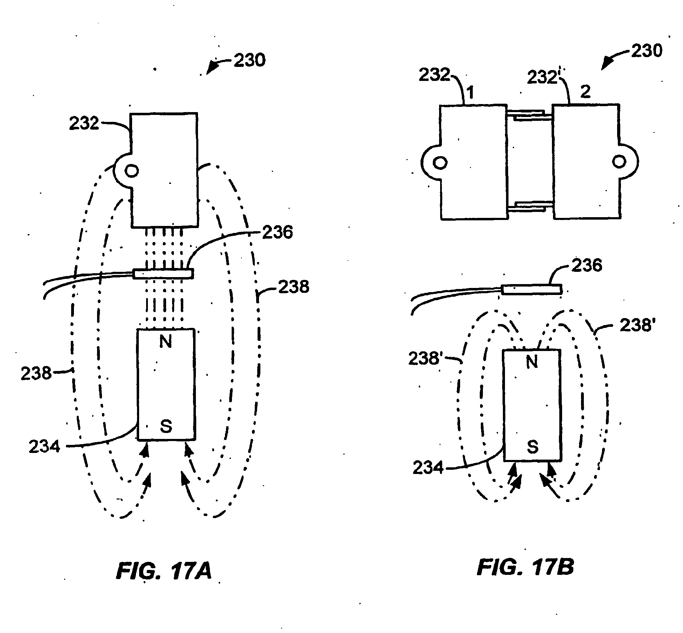

Figs. 17A and 17B show another variation for Hall sensor positioning. Fig. 17A shows a sensor assembly **230** adjacent to an individual vertebrae **232** of an endoscope. A single vertebrae **232** is shown only for the sake of clarity. As seen, when vertebrae **232** is directly adjacent to magnet **234,** magnetic flux lines **238** are disrupted and are forced to pass through sensor **236.** Flux lines **238** passing through sensor **236** may cause a disruption in the current flowing therethrough and may thus indicate the passage of the endoscope. Fig. 17B shows the assembly of Fig. 17A when endoscope **230** has been advanced or withdrawn fractionally such that magnet **234** is positioned inbetween adjacent vertebrae **232** and **232'.** When a vertebra is not immediately adjacent to magnet **234,** flux lines **238'** may return to their normal undisturbed state such that sensor **236** is also undisturbed by magnetic flux. The resumption of current within sensor **236** may indicate that endoscope **230** has been moved relative to sensor assembly **230.**

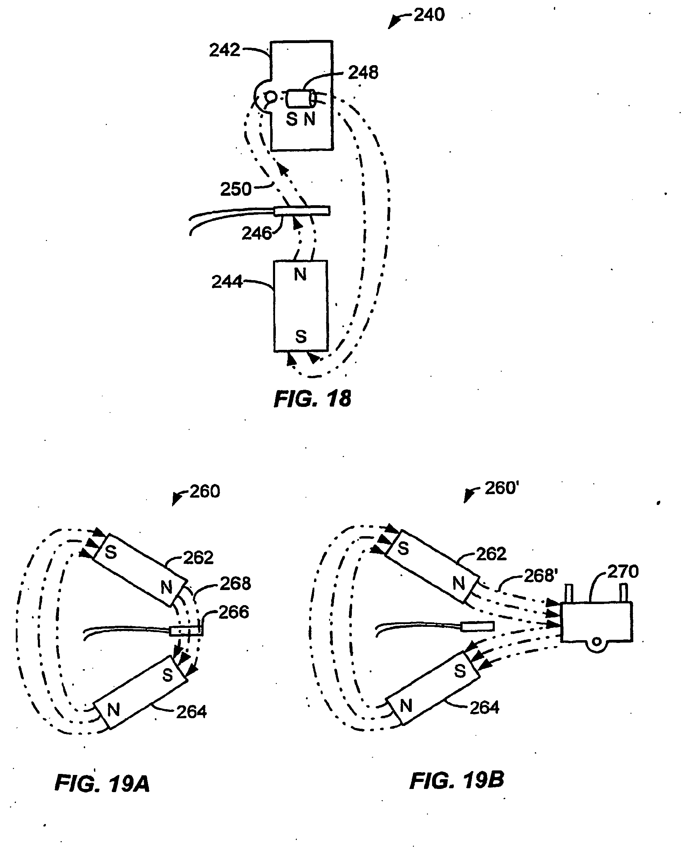

Fig. 18 shows another variation in assembly **240** where a discrete magnet **248** may be positioned on individual vertebrae **242** to produce a more pronounced effect in sensor measurement. Magnets **248** may be positioned along the longitudinal axis of the endoscope for creating a uniform magnetic field radially about the endoscope. Discrete magnets **248** may be permanent magnets or they may alternatively be electromagnets. In either case, they may be placed on as many or as few vertebrae or at various selected positions along the endoscope body depending upon the desired measurement results. As shown, when vertebrae **242** having discrete magnet **248** mounted thereon is brought into the vicinity of magnet **244,** the interaction between the magnets produces an enhanced flux interaction **250** such that Hall sensor **246** is able to sense a more pronounced measurement. The polarity of each individual magnet **248** located along the endoscope body may be varied from location to location but the polarity of adjacent magnets on the endoscope body are preferably opposite to one another.

Alternatively, a number of magnets each having a unique magnetic signature may be placed at predetermined positions along the length of the endoscope. Each magnet **248** may be mapped to its location along the endoscope so when a magnet having a specific magnetic signature is detected, the insertion depth of the endoscope may be correlated. The magnets **248** may have unique magnetic signatures, e.g., measurable variations in magnetic field strength, alternating magnetic fields (if electromagnets are utilized), reversed polarity, etc.

Figs. 19A and 19B show yet another variation in assembly **260** in which more than one magnet may be used in alternative configurations. A first magnet **262** may be positioned at an angle relative to a second magnet **264** such that the combined flux lines **268** interact in accordance with each magnet. Thus, the polarity of each magnet **262, 264** may be opposite to one another as shown in the figures. Sensor **266** may be positioned such that the undisturbed field lines **268** pass through sensor **266.** As vertebrae **270** is passed adjacent to sensor **266,** the disturbed flux lines **268',** as shown in assembly **260'** in Fig. 19B, may be altered such that they no longer pass through sensor **266** due to the interaction with vertebrae 270. Alternatively, the field lines **268** passing through sensor **266** may be altered in strength as vertebrae **270** passes.

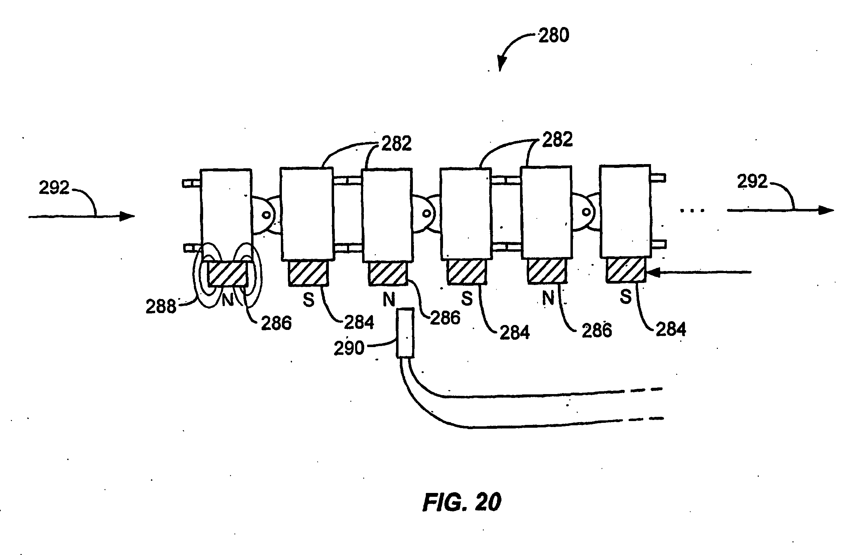

Fig. 20 shows yet another variation in which discrete magnets may be placed on each individual vertebrae of an endoscope assembly. As shown, sensor assembly **280** shows only the vertebrae **282** of an endoscope for clarity. Discrete magnets **284** having a first orientation may be placed on alternating vertebrae **282** while magnets **286** having a second orientation may be placed on alternating vertebrae **282** inbetween magnets **284.** Thus, when the endoscope is moved, e.g., along the direction of travel **292,** flux lines **288** having alternating directions on each vertebrae **282** can be sensed by sensor **290.** The measured alternating flux lines may be used as an indication of endoscope movement in a first or second direction. Each of the magnets may be positioned along the periphery of the vertebrae on a single side; however, they may also be positioned circumferentially, as described below in further detail.

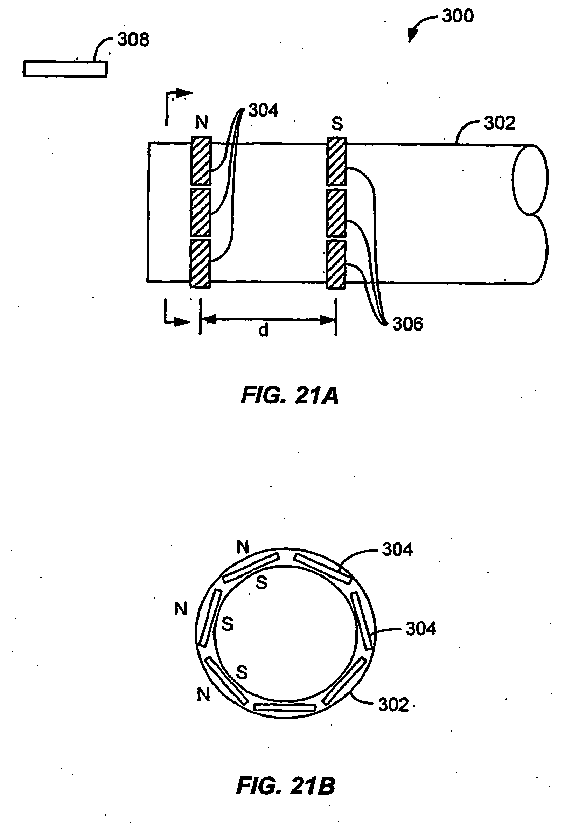

Figs. 21A and 21B show side and cross-sectional views, respectively, of another alternative in magnet positioning. Fig. 21A shows a side view of endoscope assembly **300** in which a number of magnets **304** having a first orientation may be positioned circumferentially about endoscope **302.** A number of magnets 306 having a second orientation opposite to the first orientation may also be positioned circumferentially about endoscope **302** separated a distance, d, longitudinally away from magnets **304.** With discrete magnets positioned circumferentially about endoscope **302,** the rotational orientation of endoscope **302** becomes less important as it passes sensor **308** in determining the insertion depth of the device. Fig. 21B shows a cross-sectional view of the device of Fig. 21A and shows one example of how magnets **304** may be positioned about the circumference. Although this variation illustrates magnets 304 having a "N" orientation radially outward and a "S" orientation radially inward of endoscope **302,** this orientation may be reversed so long as the adjacent set of circumferential magnets is preferably likewise reversed. Moreover, although seven magnets are shown in each circumferential set in the figure, any number of fewer or more magnets may be used as practicable.

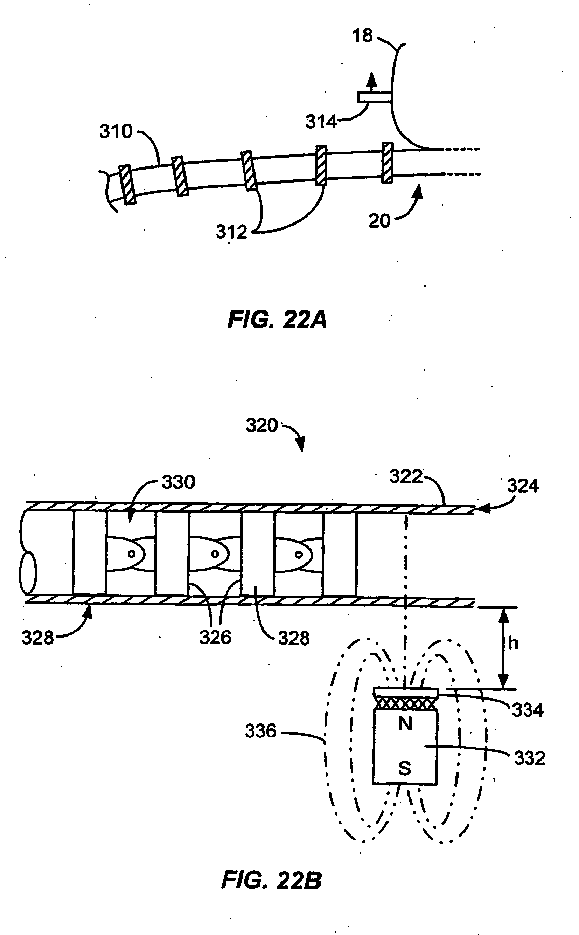

Fig. 22A shows yet another variation in which endoscope **310** may have discrete circumferentially positioned magnets **312** placed at each vertebrae 312 on an outer surface of the endoscope **310.** As endoscope **310** is passed into anus **20,** Hall sensor **314** may be positioned adjacent to anus **20** such that sensor **314** is able to read or measure the discrete magnets **312** as they pass into anus **20.** Fig. 22B shows yet another variation in which endoscope assembly **320** may have endoscope **322** in which individual vertebrae **326** may have some ferromagnetic material **328** integrated or mounted onto or within the vertebrae **326.** The ferromagnetic material **328** may be in the form of a band, coating, or other non-obstructive shape for integration onto vertebrae **326** or for coating over portions of vertebrae **326.** A sheath or skin **324** may be placed over the vertebrae **326** to: provide for a lubricious surface. Inbetween vertebrae **326,** non-magnetic regions 330 may be maintained to provide for the separation between vertebrae **326** and between ferromagnetic material **328.** Moreover, ferromagnetic material **328** may be applied retroactively not only to endoscopes having vertebrae, but also other conventional endoscopes for which a determination of insertion depth is desired. As endoscope **322** passes magnet **332,** sensor **334** may detect disturbances in flux lines **336** as the regions having the ferromagnetic material **328** passes. Additionally, endoscope **322** may be passed at a distance, h, from sensor **334** which is sufficiently close to enable an accurate measurement but far enough away so as not to interfere with endoscope **322** movement.

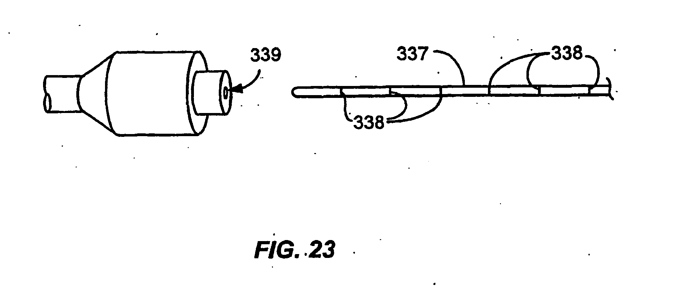

Fig. 23 shows yet another variation in which conventional endoscopes may be used with any of the Hall sensor datum devices described herein. As shown, elongate support or tool **337** may have a number of magnets 338, or ferrous material or other materials that may alter or affect a magnetic field, positioned along the tool at predetermined intervals. Magnets **338** may be positioned along the length of tool **337** such that the adjacent magnets are either alternating in polarity or uniform in polarity. Furthermore, magnets **338** may be made integrally within the tool 337 or they may be made as wireforms or members which may be crimped about tool **337.** Tool **337** may be positioned within the working lumen **339** of any conventional endoscope for use with a datum device as described herein. The inclusion of the tool **337** may then enable the determination of insertion depth of a conventional or instrumented endoscope. If a conventional endoscope is used, tool **337** may be securely held within the working lumen **339** during an exploratory procedure. Tool **337** may optionally be removed during a procedure to allow for the insertion of another tool and then reinserted within lumen **339** at a later time to proceed with the insertion and/or withdrawal of the endoscope.

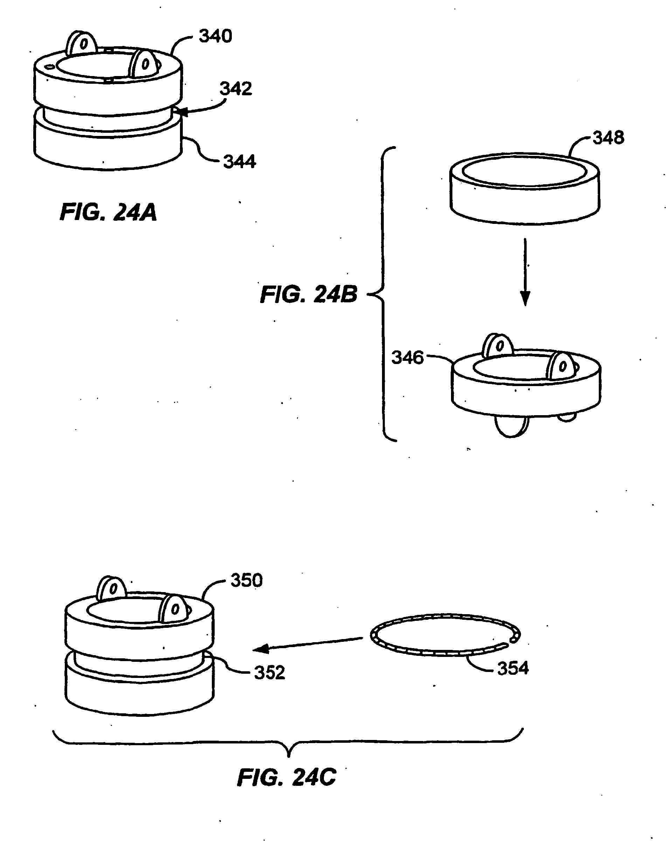

Figs. 24A to 24C show perspective views of alternative variations for attaching permanent magnets, ferrous materials, or other materials that may alter or affect a magnetic field, onto individual vertebrae. Fig. 24A shows one variation in which vertebrae **340** may be manufactured with a notch or channel **342** circumferentially defined along its outer surface **344.** A ring made of a ferrous material or other material that may alter or affect a magnetic field, such as permanent magnets, may be placed within notch **342.** Fig. 24B shows another variation in which a formed ring **348** made of a permanent magnet or other such materials may be separately formed and attached onto vertebrae **346.** Fig. 24C shows yet another variation in which a wire form **354** made from a ferrous material or other material that may alter or affect a magnetic field, such as a permanent magnet, may be placed within notch **352** of vertebrae **350.** Alternatively, ferrous powder may be molded into a circumferential shape and placed within notch **352.** Another alternative may be to simply manufacture the entire vertebrae from a ferrous metal or simply cover a vertebrae or a portion of the vertebrae with a ferrous coating.

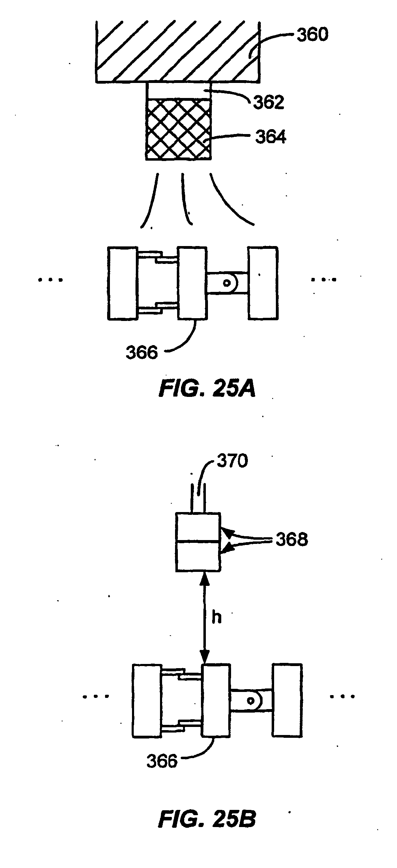

Another alternative for utilizing Hall sensors is seen in Figs. 25A and 25B. The variation in Fig. 25A may have a fixed platform **360** upon which a magnet **364** may be mounted. A pressure sensor or microforce sensor **362** may be placed inbetween magnet **364** and platform **360.** As an endoscope is passed adjacent to magnet **364,** the magnet **364** may be attracted to vertebrae **366** as it passes adjacently. Vertebrae **366** may optionally include ferrous materials or other materials that may alter or affect a magnetic field as described above to enhance the attraction and/or repulsion. As magnet **364** is pulled or repulsed by the magnetic force, pressure sensor **362** may record the corresponding positive or negative force values for correlating to endoscope insertion depth. Fig. 25B shows another example in which magnets **368** may be attached to a pressure gauge **370,** e.g., a Chatillon® gauge made by Ametek, Inc. As the endoscope passes magnets **368** at some distance, h, the attraction and/or repulsion between magnets **368** and vertebrae **366** may be accordingly measured by gauge **370** and similarly correlated to endoscope insertion depth.

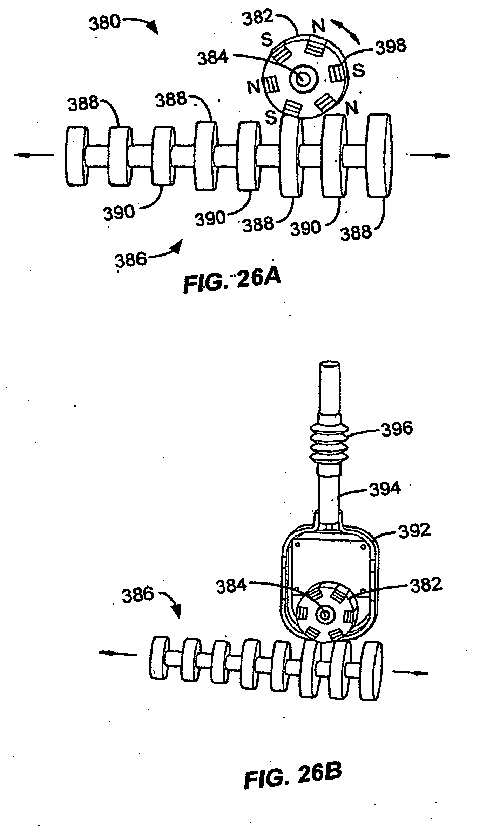

Yet another variation is shown in Figs. 26A and 26B in assembly 380. Rather than utilizing the linear motion of an endoscope past a static datum, a rotatable datum **382** may be used to record insertion length. Datum wheel **382** may be configured to rotate about pivot **384** while sensing the movement of endoscope **386,** which shows only schematic representations of the vertebrae for clarity. The datum wheel **382** may have a number of magnets **398** incorporated around the circumference of wheel **382.** Each magnet may be arranged in alternating pole configurations or alternatively in the same pole arrangement. Each of the magnets **398** are also preferably spaced apart from one another at intervals equal to the linear distances between the magnets **388, 390** or permanent magnet located along the body of endoscope **386.** Ferrous materials, or materials that may otherwise alter a magnetic field, may be used in place of the permanent magnets. As endoscope **386** is moved past datum wheel **382,** wheel **382** rotates in corresponding fashion with the linear movement of endoscope **386** past the datum 382.

The rotation of datum wheel **382** that results when endoscope **386** is moved past can be sensed by a variety of methods. One example includes rotary optical encoders, another example includes sensing the movement of magnets **398** on datum wheel **382** as they rotate relative to a fixed point as measured by, e.g., Hall effect sensors or magnetoresistive sensors. As datum wheel **382** rotates with the linear movement of endoscope **386,** datum wheel **382** may directly touch endoscope **386** or a thin material may separate the wheel **382** from the body of endoscope **386.** Fig. 26B shows one variation of an assembly view of datum wheel **382** which may be rotatably attached to housing **392.** Housing **392** may be connected to stem or support **394,** which may extend from housing **392** and provide a support member for affixing datum wheel **382** to the patient, an examination table, a stand, or any other platform. Support **394** may also be used to route any cables, wires, connectors, etc., to housing **392** and/or datum wheel **382.** The associated sensors and various support electronics, e.g., rotary encoders, magnetic field sensors, etc., may also be located within housing **392.** Support **394** may further include an optional flexible joint **396** to allow datum wheel **382** to track the movement of endoscope **386** as it passes into or out of a patient.

### EXAMPLES OF EXTERNAL SENSING DEVICES

The external sensing devices, or datum, may function in part as a point of reference relative to a position of the endoscope and/or patient, as described above. The datum may accordingly be located externally of the endoscope and either internally or externally to the body of the patient. If the patient is positioned so that they are unable to move with any significant movement during a procedure, the datum may function as a fixed point of reference by securing it to another fixed point in the room, e.g., examination table, procedure cart, etc. Alternatively, the datum may be attached directly to the patient in a fixed location relative to the point of entry of the endoscope into the patient's body. The datum variations described herein may utilize any of the sensing and measurement methods, which are not part of the invention, described above.

For instance, for colonoscopic procedures the datum may be positioned on the patient's body near the anus. The location where the datum is positioned is ideally a place that moves minimally relative to the anus because during such a procedure, the patient may shift position, twitch, flex, etc., and disturb the measurement of the endoscope. Therefore, the datum may be positioned in one of several places on the body.

One location may be along the natal cleft, i.e., the crease defined between the gluteal muscles typically extending from the anus towards the lower back. The natal cleft generally has little or no fat layers or musculature and does not move appreciably relative to the anus. Another location may be directly on the gluteal muscle adjacent to the anus.

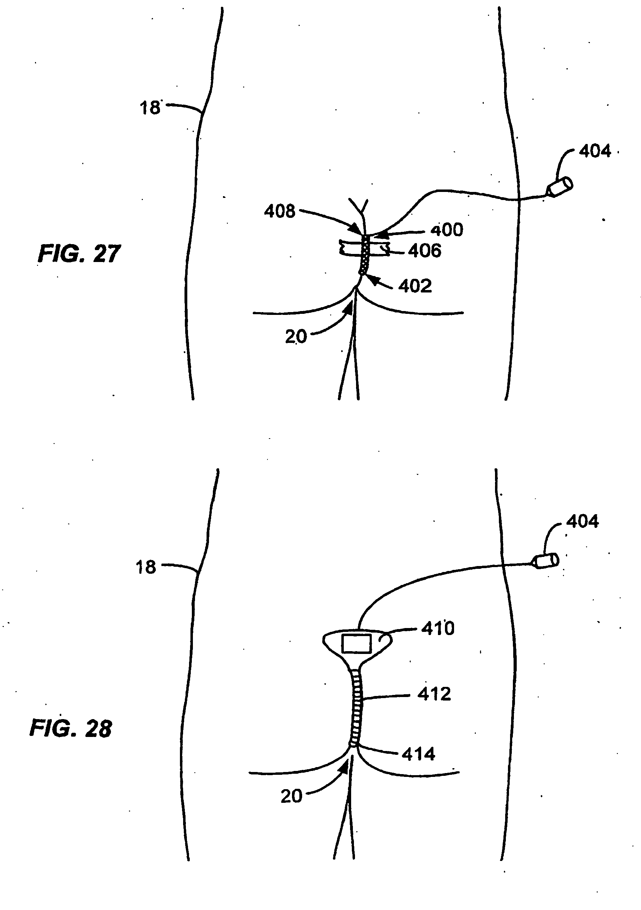

One variation for the datum for positioning along the natal cleft 408 is shown in Fig. 27. Datum **400** may have sensor **402** positioned in the distal tip of the sensing device, which may be placed adjacent to anus 20. The.datum itself may be positioned within the natal cleft 408 and temporarily held in place on the patient with adhesive **406.** The datum may have a connector **404** extending via a wire or cable for connection to a processor (not shown).

Another variation is shown in Fig. 28 in which the datum **410** may have a base comprising a substrate. The substrate may have an adhesive side that may be placed against the small of the patient's back. An elongate flexible member or arm **412** may extend from the substrate and lie within or against the natal cleft such that the distal end **414** of member **412** is adjacent to anus **20.** Distal end **414** may have a sensor mounted within for sensing the movement of an endoscope as it is passed through anus **20.** The flexible member **412** may be secured along the natal cleft using, e.g., adhesive tape, to prevent excessive movement of the device.

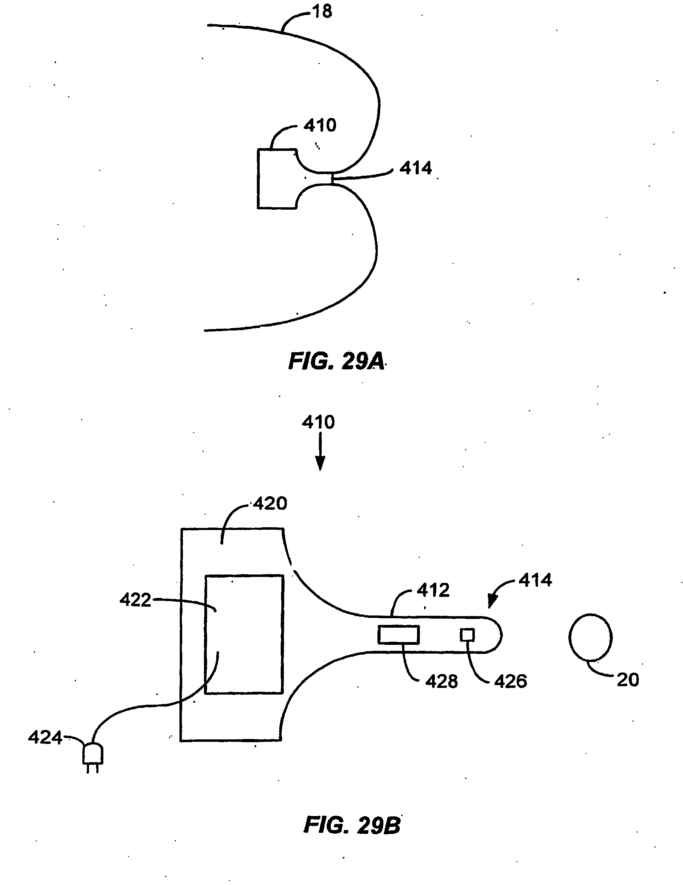

Figs. 29A and 29B show a detailed view of a variation of the datum device **410** of Fig. 28. Fig. 29A shows another view for possible positioning of datum **410** on patient **18.** The substrate may be positioned proximal of anus **20** while member **412** extends along the natal cleft for positioning sensor tip **414** proximally adjacent to anus **20.** Fig. 29B shows datum **410** laid out and having a substrate **420** upon which sensors and electronics may be positioned. Substrate **420,** as mentioned above, may have an adhesive backing for temporary placement against the patient 18. Moreover, datum **410,** or any of the other datum examples described herein, may be optionally configured to be disposable for one-time use on a patient. Support electronics **422** may optionally be placed upon substrate **420** and sensor **426** may be positioned within the distal end 414 at or near the end of the flexible member or arm **412.** An optional magnet **428** may be positioned along member **412** proximally of sensor **426.** Connector **424** may extend via a wire or cable from datum **410** for connection to a processor.

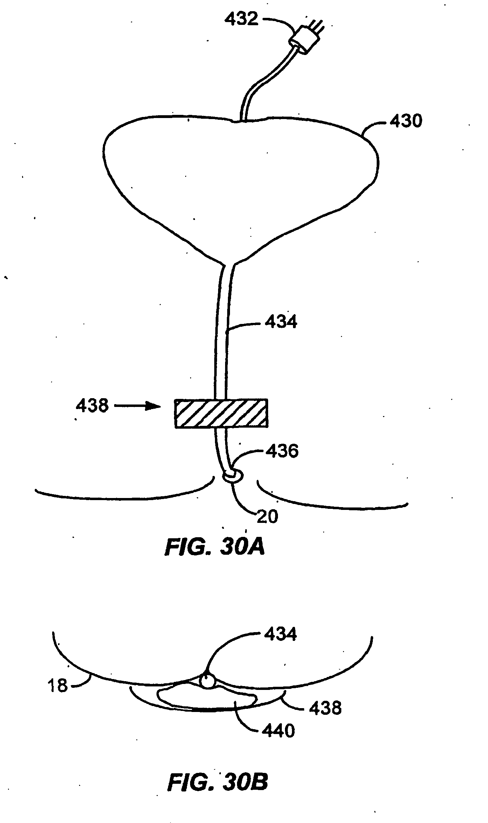

Another variation is shown in Figs. 30A and 30B which shows datum substrate **430** having sensor **436** positioned within the distal end of elongate flexible assembly **434** for placement adjacent to anus **20.** Connector **432** may be provided for connection to a processor. Here, elongate assembly **434** may be secured against or within the natal cleft by use of, e.g., an adhesive strip **438.** Fig. 30B shows a cross-sectional top-down view of elongate assembly **434** positioned against the natal cleft. A sponge, silicone wedge, or some other wedging device **440** may be positioned inbetween elongate assembly **434** and adhesive strip **438** to ensure secure positioning of the datum device relative to anus **20.**

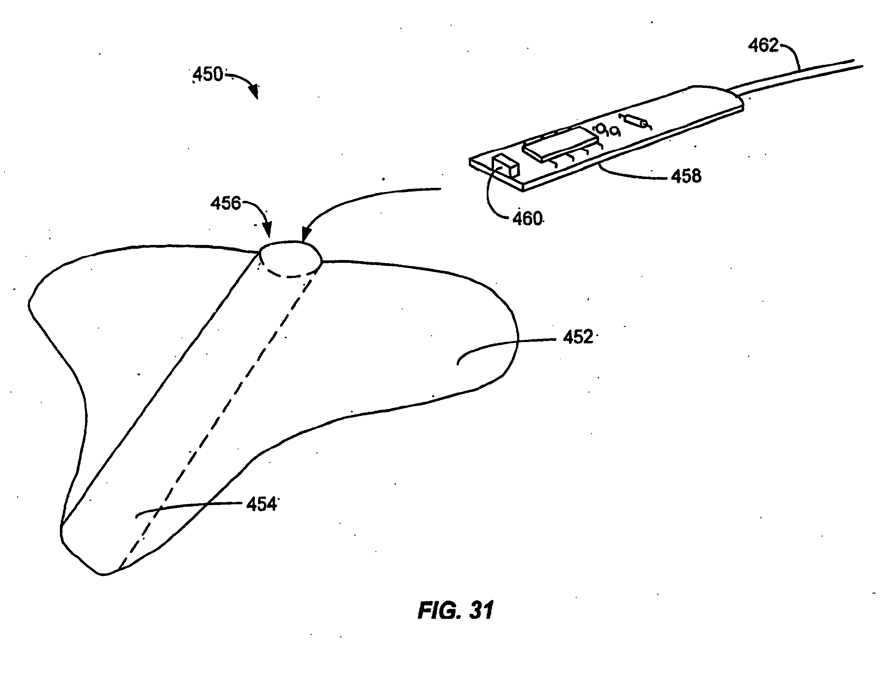

Fig. 31 shows another variation on the datum device which may utilize a disposable substrate. Datum assembly **450** may have substrate **452** for placement against the patient. A retaining pocket **454** may be defined within or upon substrate **452** and it may be configured to allow for a reusable electronic sensor assembly **458** to be placed within pocket **454.** Sensor assembly **458** may have a wire or cable **462** extending therefrom and it may further have a sensor **460** positioned or potted upon sensor assembly **458.** The sensor assembly **458** may be positioned within pocket **454** by slipping sensor assembly **458** through an opening **456** defined within substrate **452** and sensor assembly **458** is preferably positioned within pocket **454** such that sensor **460** is positioned at the distal end of substrate **452** to allow for positioning adjacent the anus.

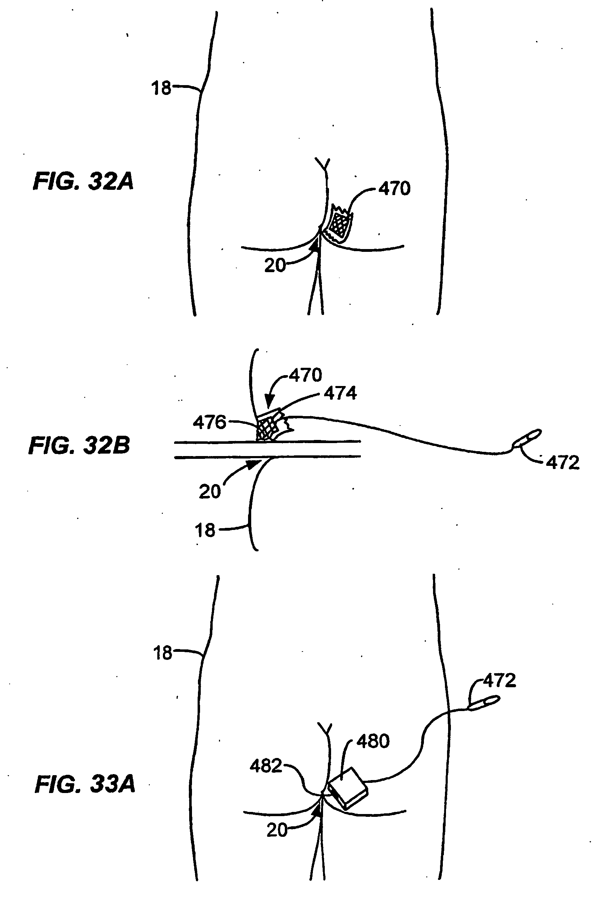

Another variation for positioning a datum is directly on the gluteal muscle adjacent to the anus. Generally, the sensor and associated circuitry may be incorporated into a patch or small chassis that may then be attached to the muscle adjacent to the anus. The entire datum assembly may optionally be mounted onto a bandage-like package with an adhesive backing. Figs. 32A and 32B show a variation in datum **470** which is formed into a small chassis having connector **472** extending therefrom. Datum **470** may be attached temporarily to patient **18** via adhesive **474** adjacent to anus **20.** A guide, ramp, or other similar structure **476** for situating, orienting, or guiding endoscope relative to datum **470** may be optionally incorporated into the device.

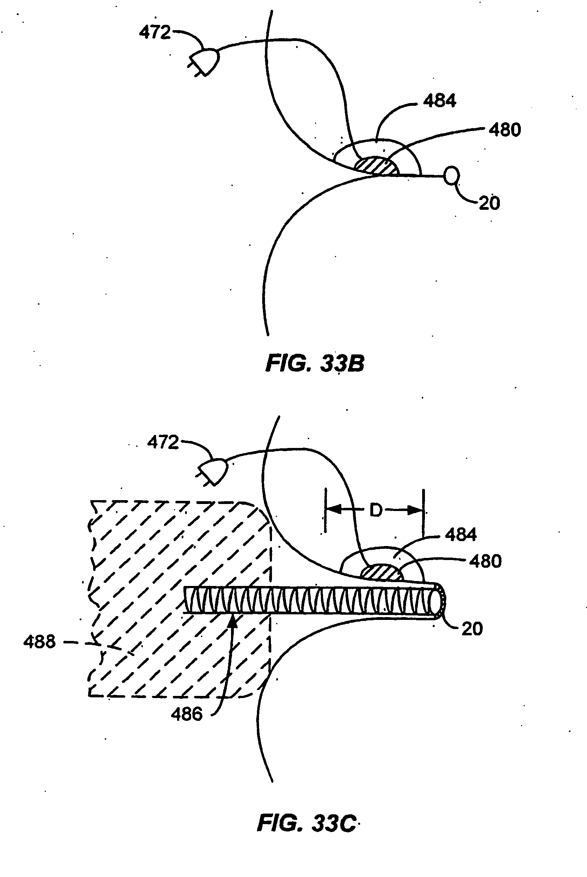

Fig. 33A shows another variation of the device in datum **480.** In this example, datum **480** may be in the form of a patch with sensor **482** positioned thereon. The device may be placed upon one of the gluteal muscles such that sensor **482** is adjacent to anus **20.** Fig. 33B shows a detailed view of how datum **480** may be positioned upon the gluteal muscle adjacent to anus **20.** Adhesive **484** may be placed over datum **480** to temporarily hold it onto the gluteal muscle as shown. Fig. 33C shows an example of how datum **480** may interact with endoscope **486** as it is advanced or withdrawn from anus **20.** Because datum **480** may have a relatively small diameter, D, discomfort may be reduced for the patient and close proximity to anus **20** may be assured. As endoscope **486** moves past datum **480,** the sensors within datum **480** may measure the insertion depth. Zone **488** shows generally the zone of operation, i.e., the region within which the operator's or surgeon's hands generally operate during a colonoscopy procedure. Because of the small diameter of datum **480** and its position adjacent anus **20,** it is generally out of the way of the operator or surgeon during a procedure and thereby allows for unhindered operation of the endoscope **486** while maintaining accurate measurement or sensing with datum **480.**

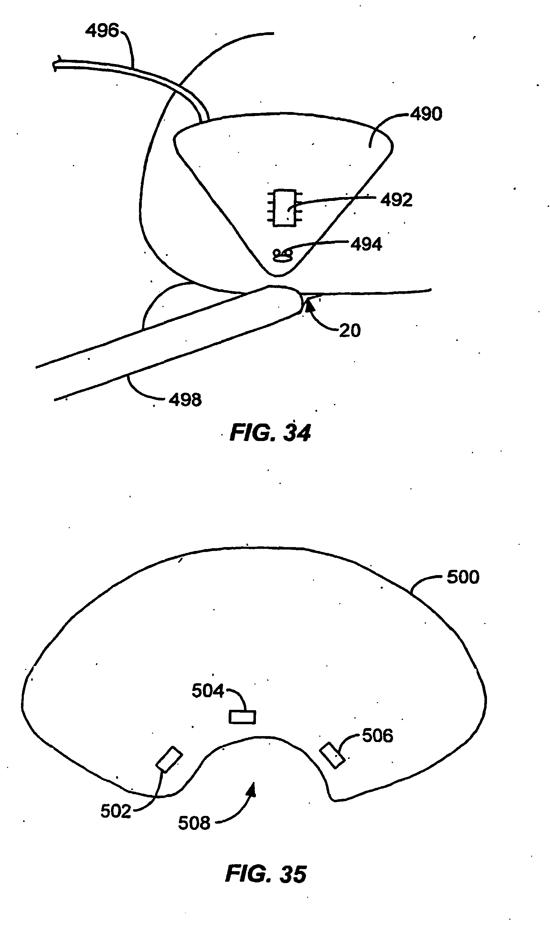

Fig. 34 shows yet another variation in datum **490** which may have a substrate with sensor **494** mounted at one end. Support electronics **492** may be optionally mounted on datum **490** and wire or cable **496** may be used to transmit the measured signals from sensor **494.** Datum **490** may be in a triangular shape for placement upon a single gluteal muscle, as shown, such that a vertex of the substrate is positioned adjacent to anus **20** to allow sensor **494** to sense or measure signals as endoscope **498** is advanced or withdrawn into anus **20.** Although shown in this variation in a triangular pattern, this is not intended to be limiting and is intended merely to illustrate one possible shape for the datum.

Another variation is shown in Fig. 35 in which datum **500** may incorporate multiple sensors. Datum **500** may be placed on a single gluteal muscle and it may define an insertion region **508** at which the anus of the patient may be positioned. Each of the sensors **502, 504, 506** may thereby be configured to sense or read the endoscope as it passes through or past the insertion region **508.** Although three sensors are shown in this configuration, fewer or more sensors may be utilized depending upon the configuration of the datum **500** and the desired signal processing results.

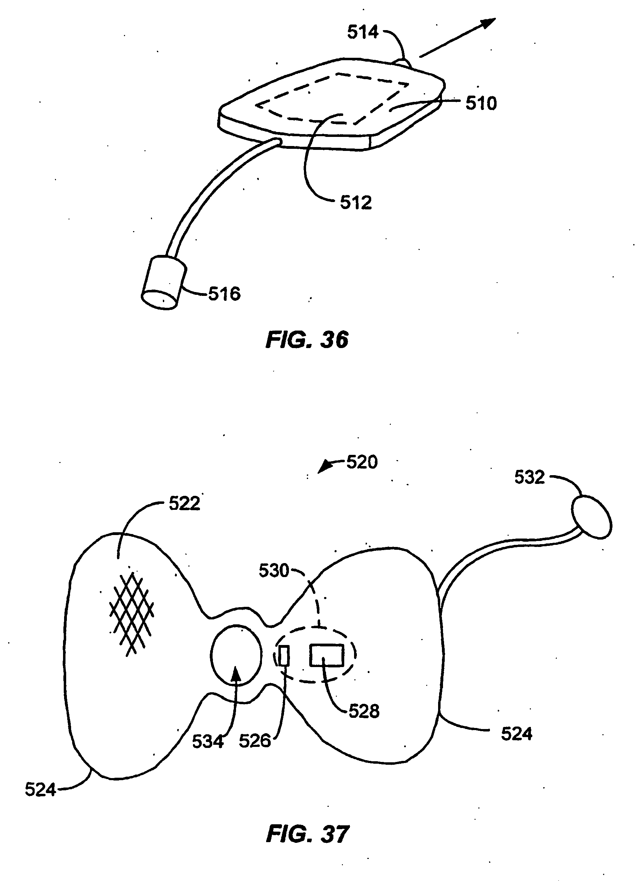

Fig. 36 shows yet another variation in which datum **510** may be encased in a rigid housing. Datum **510** may thus encapsulate support electronics 512 within with sensor **514** directed towards one end of the housing. The housing may incorporate a connector **516** attached via a wire or cable extending from the datum **510.** The rigid housing may be temporarily adhered to the patient on a gluteal muscle in the same fashion as described above.

Fig. 37 shows yet another variation in which datum **520** may be configured to extend across the natal cleft to position an opening defined in the datum over the anus of the patient. As shown, an adhesive substrate **522** may be configured, e.g., into a "butterfly" configuration. Substrate **522** may have at least two wings or flaps **524** for adhering to each gluteal muscle across the natal cleft while sensor **526** and support electronics **528** may be contained adjacent an opening **534** defined at or near the center of substrate **522.** Sensor **526** and support electronics **528** may be potted or contained within a housing **530** on substrate **522.** Connector **532** may be attached via a wire or cable for connection to a processor.

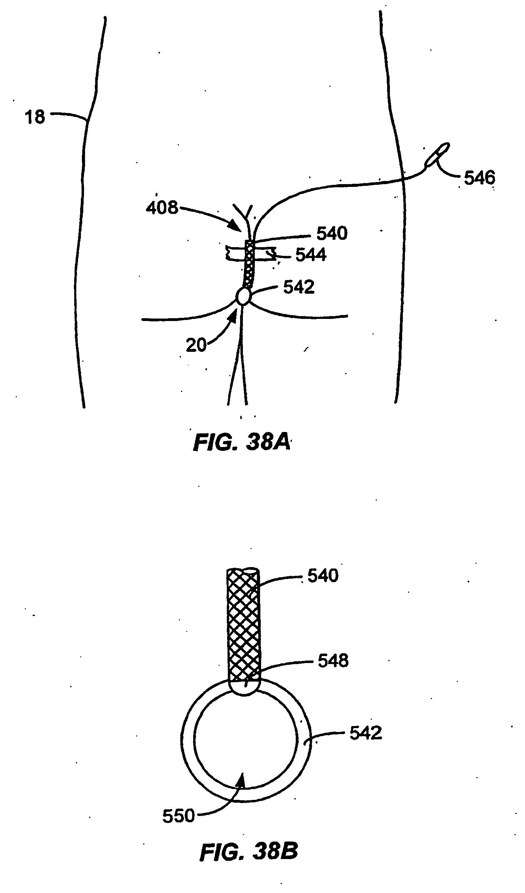

A datum device may also be configured to encircle an endoscope as it passes into the body. Such a datum configuration may be useful when using sensing technology such as RF. In the case of RF, the datum may be in a looped configuration to facilitate the exchange of RF signals with components or sensors mounted along the endoscope, as described above. One variation of a looped datum configuration is shown in Figs. 38A and 38B. As shown, datum 540 may have a loop **542** defined at a distal end to function as a signal receiver, e.g., RF signals, and/or as a guide loop. The datum **540** may be aligned along the natal cleft **408** and secured in place with adhesive tape **544.** A connector **546** may be attached to datum **540** via a wire or cable at a first end of datum **540** while sensor **548** may be positioned at the opposing end of datum **540.** Sensor **548** may be positioned adjacent to anus **20,** while loop **542** encircles the opening of anus **20.** The loop **542** may define an insertion region **550** through which an endoscope may be passed. The loop **542** may be made of a,thin, flexible material such as mylar and it optionally have an adhesive backing for placement upon the tissue surrounding anus **20.** Although shown in a circle configuration, loop **542** may be in a variety of looped configuration and is not limited by its shape.

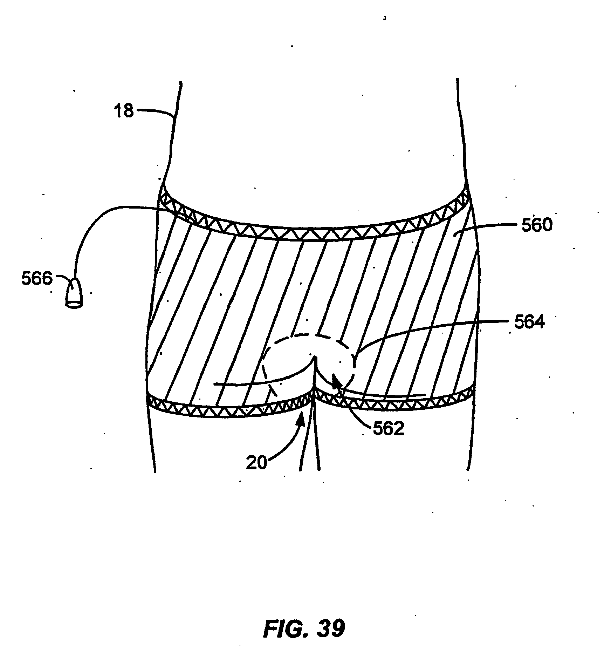

Yet another variation is shown in Fig. 39 where a supporting garment **560,** e.g., a pair of underpants, may define an opening **562** in the region surrounding the anus **20.** A loop **564** may be incorporated into the fabric such that the loop surrounds the opening **562.** The fabric in the middle of loop **564** may either be removable at the time of the procedure or omitted altogether. Connection to the loop **564** may be made through connector **566,** which can be connected via a wire or cable extending from, e.g., the waistband, front, or side of garment **560.**

Aside from colonoscopy, other applications may include uses in minimally invasive surgery (MIS). MIS typically depends upon the use of long, thin tools for insertion into the body via small incisions, e.g., often through a cannula. Instruments typically employed during MIS may include rigid endoscopes, laparoscopes, thoracoscopes, needle drivers, clamps, etc. Because each of these tools must pass through an opening in the body, a datum device may be used adjacent to that body opening for tracking instrument insertion depth. In situations where cannulas are used, the cannula itself may be instrumented through one of the methods described above.

For other types of endoscopy procedures, various types of flexible endoscopes may be used, e.g., upper endoscopes, duodenoscopes, sigmoidscopes, bronchoscopes, neuroscopes, ENT scopes, etc. Any of the devices and methods, which are not part of the invention, described above may be utilized and configured to maintain insertion depth for any of these types of endoscopes. For instance, for flexible endoscopes that enter the body transorally, a mouthpiece configured as a datum may be utilized.

The applications of the devices and methods, which are not part of the invention, discussed above are not limited to regions of the body but may include any number of further treatment applications. Other treatment sites may include other areas or regions of the body. Additionally, the present invention may be used in other environments such as exploratory procedures on piping systems, ducts, etc. Modification of the above-described assemblies and methods, which are not part of the invention, for carrying out the invention, and variations of aspects of the invention that are obvious to those of skill in the art are intended to be within the scope of the claims.

## Claims

1. A system to determine a depth of insertion of an endoscopic device (92, 112, 122, 208, 300) with reference to an anatomical landmark, the system comprising
a plurality of sensible tags (94, 114, 124, 304, 306) positioned along a length of at least a portion of the endoscopic device;
a sensing device (96, 116, 126, 130, 132, 134, 150, 152, 200, 204, 206, 308) that is external to the endoscopic device, wherein a reader (98, 118) in the sensing device senses information associated with each tag as each tag comes close to the reader when the endoscopic device is moved past the sensing device;
a system constructed and arranged to record and store information associated with the sensing of each tag; and
an insertion depth determining system constructed and arranged to determine the depth of insertion of the endoscopic device with reference to the anatomical landmark based on a position of the sensing device with reference to the anatomical landmark and based on the recorded and stored information associated with the most recently sensed tag.

2. The system of claim 1, wherein a first one of the tags comprises a first plurality of magnets (304) positioned around a first circumference of the endoscopic device (300) in a first orientation, and wherein a second one of the tags comprises a second plurality of magnets (306) positioned around a second circumference of the endoscopic device in a second orientation that is different from the first orientation.

3. The system of claim 1, wherein each tag comprises a material that alters a magnetic field, wherein the reader comprises a magnet coupled to a pressure sensor, and wherein the sensed information associated with each tag as each tag comes close to the reader comprises an attraction or repulsion of the magnet in response to the material that alters a magnetic field as sensed by the pressure sensor.

4. The system of claim 1, wherein the reader comprises an optical sensor.

5. The system of claim 1, wherein the plurality of sensible tags comprise surface features of the endoscopic device, and wherein the reader comprises an imaging system that detects the surface features as the endoscopic device is moved past the sensing device.

6. The system of claim 1, wherein each tag in the plurality of tags causes the reader to sense a value that is unique to each tag.

7. The system of claim 1, wherein each tag comprises a magnet having a magnetic signature that is unique.

8. The system of claim 1, wherein the tags (94) are selected from the group consisting of RF identification tags, ultrasonic tags, passive encoded markers, active encoded markers, IR tags, and magnetic tags.

9. The system of claim 1, wherein the reader comprises at least one pressure sensor, and wherein the tags comprise features on the endoscopic device that activate the at least one pressure sensor.

10. The system of claim 1, wherein each tag comprises a change in diameter (102, 104, 106) of the endoscopic device (112).

11. The system of claim 1, further comprising:
a second sensing device that is external to the endoscopic device, wherein a second reader in the second sensing device senses information associated with each tag as each tag comes close to the second reader when the endoscopic device is moved past the second sensing device; and
a direction of movement determining system that determines a direction of movement of the endoscopic device by comparing information associated with a particular tag sensed by the reader with information associated with the particular tag sensed by the second reader.

12. The system of claim 11, wherein the information associated with the sensing of the particular tag by the reader and the information associated with the sensing of the particular tag by the second reader comprises a change in a voltage.

## Patentansprüche

1. System zur Bestimmung der Einführtiefe einer endoskopischen Vorrichtung (92, 112, 122, 208, 300) bezüglich eines anatomischen Orientierungspunkts, wobei das System enthält:
eine Vielzahl von tastbaren Markierungen (94, 114, 124, 304, 306), die entlang einer Länge mindestens eines Abschnitts der endoskopischen Vorrichtung positioniert sind;
eine Abtastvorrichtung (96, 116, 126, 130, 132, 134, 150, 152, 200, 204, 206, 308), die sich außerhalb der endoskopischen Vorrichtung befindet, wobei ein Lesegerät (98, 118) in der Abtastvorrichtung die jeder Markierung zugeordnete Information abtastet, während jede Markierung sich dem Lesegerät nähert, wenn die endoskopische Vorrichtung an der Abtastvorrichtung vorbei bewegt wird;
ein System, das gestaltet und angeordnet ist, um eine mit dem Abtasten jeder Markierung verbundene Information aufzuzeichnen und zu speichern; und
ein System zur Bestimmung der Einführtiefe, das gestaltet und angeordnet ist, um die Einführtiefe der endoskopischen Vorrichtung bezüglich des anatomischen Orientierungspunkts basierend auf einer Stellung der Abtastvorrichtung bezüglich des anatomischen Orientierungspunkts und basierend auf der mit der zuletzt abgetasteten Markierung verbundenen aufgezeichneten und gespeicherten Information zu bestimmen.

2. System nach Anspruch 1, wobei eine erste der Markierungen eine erste Vielzahl von Magneten (304) enthält, die um einen ersten Umfang der endoskopischen Vorrichtung (300) herum in einer ersten Ausrichtung positioniert sind, und wobei eine zweite der Markierungen eine zweite Vielzahl von Magneten (306) enthält, die um einen zweiten Umfang der endoskopischen Vorrichtung herum in einer zweiten Ausrichtung positioniert sind, die sich von der ersten Ausrichtung unterscheidet.

3. System nach Anspruch 1, wobei jede Markierung ein Material enthält, das ein Magnetfeld ändert, wobei das Lesegerät einen mit einem Drucksensor verbundenen Magnet enthält, und wobei die jeder Markierung zugeordnete abgetastete Information, während jede Markierung sich dem Lesegerät nähert, eine Anziehung oder Abstoßung des Magnets als Reaktion auf das Material enthält, das ein Magnetfeld ändert, wie es vom Drucksensor abgetastet wird.

4. System nach Anspruch 1, wobei das Lesegerät einen optischen Sensor enthält.

5. System nach Anspruch 1, wobei die Vielzahl von tastbaren Markierungen Oberflächenmerkmale der endoskopischen Vorrichtung enthalten, und wobei das Lesegerät ein bildgebendes System enthält, das die Oberflächenmerkmale erfasst, während die endoskopische Vorrichtung an der Abtastvorrichtung vorbei bewegt wird.

6. System nach Anspruch 1, wobei jede Markierung der Vielzahl von Markierungen das Lesegerät dazu bringt, einen Wert abzutasten, der für jede Markierung einzigartig ist.

7. System nach Anspruch 1, wobei jede Markierung einen Magnet enthält, der eine magnetische Signatur enthält, die einzigartig ist.

8. System nach Anspruch 1, wobei die Markierungen (94) aus der Gruppe ausgewählt werden, die besteht aus RF-Identifikationsmarkierungen, Ultraschall-Markierungen, passiven codierten Markern, aktiven codierten Markern, IR-Markierungen und magnetischen Markierungen.

9. System nach Anspruch 1, wobei das Lesegerät mindestens einen Drucksensor enthält, und wobei die Markierungen Merkmale auf der endoskopischen Vorrichtung enthalten, die den mindestens einen Drucksensor aktivieren.

10. System nach Anspruch 1, wobei jede Markierung eine Durchmesseränderung (102, 104, 106) der endoskopischen Vorrichtung (112) enthält.

11. System nach Anspruch 1, das weiter enthält:
eine zweite Abtastvorrichtung, die sich außerhalb der endoskopischen Vorrichtung befindet, wobei ein zweites Lesegerät in der zweiten Abtastvorrichtung die jeder Markierung zugeordnete Information abtastet, während jede Markierung sich dem zweiten Lesegerät nähert, wenn die endoskopische Vorrichtung an der zweiten Abtastvorrichtung vorbei bewegt wird; und
ein die Bewegungsrichtung bestimmendes System, das eine Bewegungsrichtung der endoskopischen Vorrichtung durch Vergleich der einer bestimmten vom Lesegerät abgetasteten Markierung zugeordneten Information mit einer der vom zweiten Lesegerät abgetasteten bestimmten Markierung zugeordneten Information bestimmt.

12. System nach Anspruch 11, wobei die dem Abtasten der bestimmten Markierung durch das Lesegerät zugeordnete Information und die dem Abtasten der bestimmten Markierung durch das zweite Lesegerät zugeordnete Information eine Spannungsänderung enthalten.

## Revendications

1. Système pour déterminer la profondeur d'insertion d'un dispositif endoscopique (92, 112, 122, 208, 300) par rapport à un repère anatomique, le système comprenant :
une pluralité d'étiquettes sensibles (94, 114, 124, 304, 306) placées le long d'au moins une partie du dispositif endoscopique ;
un dispositif de détection (96, 116, 126, 130, 132, 134, 150, 152, 200, 204, 206, 308) qui est extérieur au dispositif endoscopique, dans lequel un lecteur (98, 118) placé dans le dispositif de détection détecte une information associée à chaque étiquette alors que chaque étiquette s'approche du lecteur lorsque le dispositif endoscopique passe devant le dispositif de détection ;
un système constitué et conçu pour enregistrer et mémoriser une information associée à la détection de chaque étiquette ; et
un système de détermination de profondeur d'insertion constitué et conçu pour déterminer la profondeur d'insertion du dispositif endoscopique par rapport au repère anatomique en fonction d'une position du dispositif de détection par rapport au repère anatomique et en fonction de l'information enregistrée et mémorisée associée à l'étiquette la plus récemment détectée.

2. Système selon la revendication 1, dans lequel une première des étiquettes comprend une première pluralité d'aimants (304) placés autour d'une première circonférence du dispositif endoscopique (300) dans une première orientation, et dans lequel une seconde des étiquettes comprend une seconde pluralité d'aimants (306) placés autour d'une seconde circonférence du dispositif endoscopique dans une seconde orientation qui est différente de la première orientation.

3. Système selon la revendication 1, dans lequel chaque étiquette comprend un matériau qui modifie un champ magnétique, dans lequel le lecteur comprend un aimant couplé à un capteur de pression, et dans lequel l'information détectée associée à chaque étiquette lorsque chaque étiquette s'approche du lecteur comprend une attraction ou répulsion de l'aimant en réponse au matériau qui modifie un champ magnétique qui est détecté par le capteur de pression.

4. Système selon la revendication 1, dans lequel le lecteur comprend un capteur optique.

5. Système selon la revendication 1, dans lequel la pluralité d'étiquettes sensibles comprend des caractéristiques de surface du dispositif endoscopique, et dans lequel le lecteur comprend un système d'imagerie qui détecte les caractéristiques de surface alors que le dispositif endoscopique passe devant le dispositif de détection.

6. Système selon la revendication 1, dans lequel chaque étiquette de la pluralité d'étiquettes amène le lecteur à détecter une valeur qui est spécifique à chaque étiquette.

7. Système selon la revendication 1, dans lequel chaque étiquette comprend un aimant ayant une signature magnétique qui est unique.

8. Système selon la revendication 1, dans lequel les étiquettes (94) sont sélectionnées parmi le groupe comprenant les étiquettes d'identification à radiofréquence, les étiquettes à ultrasons, les marqueurs codés passifs, les marqueurs codés actifs, les étiquettes infrarouges, et les étiquettes magnétiques.

9. Système selon la revendication 1, dans lequel le lecteur comprend au moins un capteur de pression, et dans lequel les étiquettes comportent des caractéristiques sur le dispositif endoscopique qui activent le au moins un capteur de pression.

10. Système selon la revendication 1, dans lequel chaque étiquette comprend un changement de diamètre (102, 104, 106) du dispositif endoscopique (112).

11. Système selon la revendication 1, comprenant en outre :
un second dispositif de détection qui est extérieur au dispositif endoscopique, dans lequel un second lecteur placé dans le second dispositif de détection détecte une information associée à chaque étiquette alors que chaque étiquette s'approche du second lecteur lorsque le dispositif endoscopique passe devant le second dispositif de détection ; et
un système de détermination de direction de mouvement qui détermine une direction de mouvement du dispositif endoscopique en comparant une information associée à une étiquette particulière détectée par le lecteur avec une information associée à l'étiquette particulière détectée par le second lecteur.

12. Système selon la revendication 11, dans lequel l'information associée à la détection de l'étiquette particulière par le lecteur et l'information associée à la détection de l'étiquette particulière par le second lecteur comprend un changement de tension.
